# EUROPEAN PATENT SPECIFICATION

(11) **EP 0 291 982 B1**
(45) Date of publication and mention of the grant of the patent: **14.07.1993**
(21) Application number: 88108048.5
(22) Date of filing: 19.05.1988
(51) Int. Cl.: C07K 7/06, C07K 7/08, A61K 37/02

(54) **Cyclic anticoagulant peptides**
Zyklisches antikoagulierendes Peptid
Peptides cycliques anticoagulants

(30) Priority: 21.05.1987 US 53169
(43) Date of publication of application: 23.11.1988
(73) Proprietor: MERRELL DOW PHARMACEUTICALS INC., Cincinnati Ohio 45215-6300 (US)
(72) Inventor: Krstenansky, John L., Cincinnati, OH 45208 (US); Mao, Simon J.T., Loveland, OH 45140 (US)
(74) Representative: VOSSIUS & PARTNER

(56) References cited:
- BIOCHIMICA ET BIOPHYSICA ACTA, vol. 957, 1988, Elsevier(US); J.L. KRSTENANSKY et al., pp. 53-59#

## Description

This invention relates to novel cyclic peptides which are useful anticoagulant agents.

Anticoagulants are useful therapeutic agents in the pharmacological treatment of, for example, acute deep venous thrombosis, pulmonary embolism, acute arterial embolization of the extremities, myocardial infarction, and disseminated intravascular coagulation. Prophylactic administration of anticoagulants is believed to prevent a recurrance of embolism in patients with rheumatic or arteriosclerotic heart disease and to prevent certain thromboembolic complications of surgery. Administration of anticoagulants has also been indicated in the treatment of coronary artery and cerebrovascular disease. Artrial thrombosis, particularly in arteries supplying the heart muscle and brain, is a leading cause of death.

Hirudin is a 65 residue polypeptide isolated from the salivary glands of leeches. It is an anticoagulant agent, which is a thrombin specific inhibitor. Although quite potent, clinical use of hirudin isolated from leech extracts seems unlikely because of its limited quantity, expense and allergic reactions which commonly follow administration of any foreign protein of this size.

Applicants have discovered a specific region of hirudin that is responsible, at least in part, for its anticoagulant activity. This region has been chemically synthesized and certain of its cyclic analogs appear to bind to the recognition site of thrombin but not the enzymatic cleavage site which is spatially separate. Binding of the synthetic peptides competitively prevents binding of the fibrinogen to the recognition site of thrombin, a prerequisite to fibrin production and clot formation. The peptides of this invention possess significant anticoagulant activity and their unusual ability to bind only to the recognition site without binding to the cleavage site of thrombin may allow for a scientifically interesting and therapeutically significant adjunct to anticoagulant therapy.

This invention relates to derivatives of Hirudin having the structural formula 1: 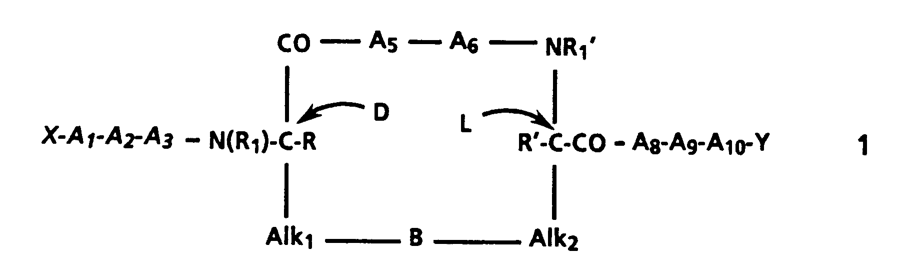
wherein
- X: is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl;
- A₁: is a bond or is a peptide containing from 1 to 5 residues of any amino acid;
- A₂: is Phe, SubPhe, β-(2- and 3-thienyl)alanine, β-(2- and 3-furanyl)alanine, β-(2-,3-, and 4-pyridyl)alanine, β-(benzothienyl-2- and 3-yl)alanine, β-(1- and 2-naphthyl)alanine, Tyr, or Trp;
- A₃: is Glu or Asp;
- A₅: is Ile, Val, Leu, Nle, or Thr;
- A₆: is Pro, Hyp, 3,4-dehydroPro, thiazolidine-4-carboxylate, Sar, NMePgl, or any amino acids having a D-configuration;
- A₈: is any amino acid;
- A₉: is a lipophilic amino acid selected from Tyr, Tyr(SO₃H), Trp, Phe, Leu, Nle, Ile, Val, His, and Pro or is a dipeptide containing at least one of these lipophilic amino acids;
- A₁₀: is a bond or is a peptide fragment containing from one to five residues of any amino acid;
- Y: is a carboxy terminal residue selected from OH, (C₁-C₆)alkoxy, amino, mono- or di-(C₁-C₄)alkyl substituted amino, or benzylamino;
- R, R', R₁, and R₁': are each selected from a hydrogen or (C₁-C₄)alkyl group;
- B: is selected from -S-, -S-S-, or -S-Alk₃-S-; and
- Alk₁, Alk₂, and Alk₃: are each selected from a (C₁-C₈)methylene or ethylene group;
and wherein "D" and "L" indicate that the stereochemistry of the indicated carbon is that corresponding to D-cysteine and L-cysteine, respectively
as well as the dimers of these peptide derivatives and their mixtures and the use of these peptide derivatives, dimers, and mixtures as anticoagulant agents.

The following common abbreviations of the amino acids are used throughout this specification:
- Gly: - glycine
- Ala: - alanine
- Val: - valine
- Leu: - leucine
- Ile: - isoleucine
- Pro: - proline
- Phe: - phenylalanine
- Trp: - tryptophan
- Met: - methionine
- Ser: - serine
- Thr: - threonine
- Cys: - cysteine
- Tyr: - tyrosine
- Asn: - asparagine
- Gln: - glutamine
- Asp: - aspartic acid
- Glu: - glutaminc acid
- Lys: - lysine
- Arg: - arginine
- His: - histidine
- Nle: - norleucine
- Hyp: - hydroxyproline
- 3,4-dehydroPro: - 3,4-dehydroproline
- Tyr(SO₃H): - tyrosine sulfate
- Pgl: - phenylglycine
- NMePgl: - N-methyl-phenylglycine
- Sar: - sarcocine (N-methylglycine)
- pSubPhe: - para substituted phenylalanine
- SubPhe: - ortho, meta, or para, mono- or di-substituted phenylalanine
- DAla: - D-alanine
- Ac: - acetyl
- Suc: - succinyl
- pClPhe: - para-chloro-phenylalanine
- pNO₂Phe: - para-nitro-phenylalanine
- Pen: - penicillamine (β,β-dimethylcysteine)
- DCys: - D-cysteine

An alkyl group and the alkyl portion of an alkoxy group is taken to include straight, branched, or cyclic alkyl groups, for example, methyl, ethyl, propyl, isopropyl, butyl, isobutyl, tert-butyl, pentyl, isopentyl, sec-pentyl, cyclopentyl, hexyl, isohexyl, cyclohexyl and cyclopentylmethyl. An acyl group of from 2 to 10 carbon atoms is taken to include straight, branched, cyclic, saturated and unsaturated acyl groups having 1 or 2 carbonyl moieties per group, for example, acetyl, benzoyl and succinyl. The term "a (C₁-C₈)methylene or ethylene group" refers to a bivalent group derived from an acyclic or cyclic, saturated or unsaturated alkyl group of from 1 to 8 carbon atoms by conceptual removal of two hydrogen atoms from one of the carbon atoms or from two of the adjacent carbon atoms of the alkyl group. Examples of the (C₁-C₈)methylene or ethylene groups of this invention are methylene or methylidene (-CH₂-), ethylidene (CH₃CH<), 1-methylethylidene (CH₃C(CH₃)<), 1-methylpropylidene or sec-butylidene (CH₃CH₂C(CH₃)<), 2,2-dimethylpropylidene or neopentylidene (CH₃C(CH₃)₂CH<), ethylene or dimethylene (-CH₂CH₂-), methylethylene (-CH₂CH(CH₃)-), ethylethylene (-CH₂CH(C₂H₅)-), ethenylene or vinylene (-CH=CH-), 1,1-ethenylidene (CH₂=C<), 1,1-cyclohexylidene (C₆H₁₀<), and 1,2-cyclopentylidene (C₅H₈<). A halogen group is a fluoro, chloro, bromo or iodo group.

The term "any amino acid" as used herein includes the naturally occurring amino acids as well as other "non-protein" α-amino acids commonly utilized by those in the peptide chemistry arts when preparing synthetic analogs of naturally occurring peptides. The naturally occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalanine (Cha), aminophenylbutyric acid (Pba), phenylalanines substituted at the para position of the phenyl moiety with a (C₁-C₄) alkyl, (C₁-C₄) alkoxy, halogen, or nitro groups, β-(2- and 3-thienylalanine, β-(2- and 3-furanyl)alanine, β-(2-, 3-, and 4-pyridyl)alanine, β-(benzothienyl-2- and 3-yl)alanine, β-(1- and 2-naphthyl)alanine, O-alkylated derivates of serine, threonine, or tyrosine, S-alkylated cysteine, the O-sulfate ester of tyrosine, 3,5-diiodotyrosine and the D-isomers of the naturally occurring amino acids.

The term "lipophilic amino acid" includes Tyr, Tyr(SO₃H), Phe, Leu, Nle, Ile, Val, His, and Pro.

The natural amino acids with the exception of glycine, contain a chiral carbon atom. Unless otherwise specifically indicated, the optically active amino acids, referred to herein, are of the L-configuration. For example, any of the amino acids of the A₁ or A₁₀ group can be of the D- or L-configuration. As is customary, the structure of peptides written out herein is such that the amino terminal end is on the left side of the chain and the carboxy terminal end is on the right side of the chain.

The term "dimers" is intended to mean those peptides which result from the linking of two seperate linear peptides during the cyclization step either in a head to head or head to tail fashion. In the course of performing the desired internal cyclization via the "B" group, some of the linear peptide starting material will link with another linear peptide starting material rather then with itself. The resulting product is a "dimer" in the sense that it is made up of two of the linear starting peptides but is not a dimer in the sense that the molecular formula of the dimer is exactly two times the molecular formula of the monomer. A dimer of the peptide derivatives of this invention will have the structural formula: 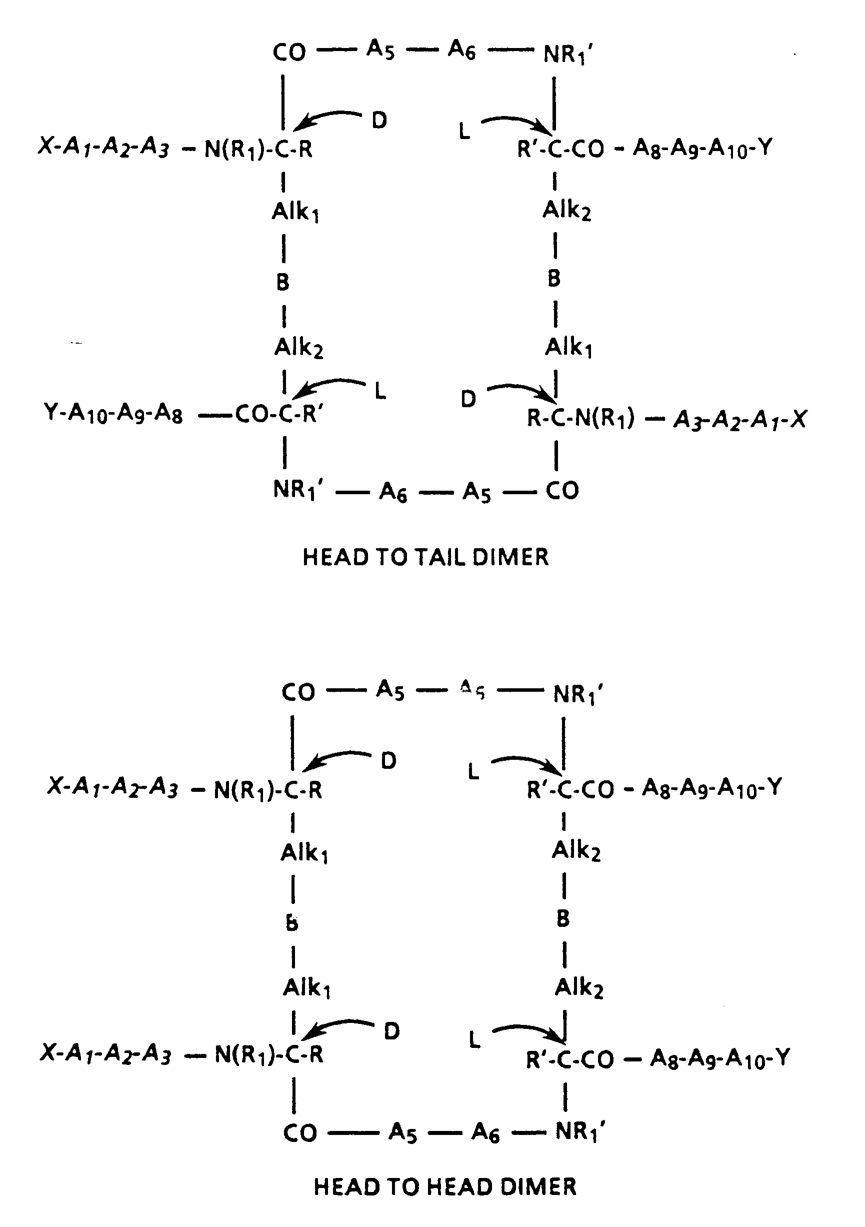
wherein the substitutents are as defined above for structure 1. Throughout this disclosure, reference to the peptide derivatives includes the dimers and mixtures unless the context requires otherwise. While the mixtures of monomer and dimer resulting from the cyclization reation can be readily seperated by means well-known to those skilled in the art, the mixtures can be utilized in the antithrombotic compositions of this invention without seperation.

The polypeptides of formula 1 can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkylamines, including triethylamine, procaine, dibenzylamine, 1-ethenamine, N,N'-dibenzylethylenediamine, dihydroabietylamine, N-(lower)alkylpiperidine, and any other suitable amine. As with any generic group of chemical compounds, certain groups are preferred. Applicants prefer those peptide derivatives of formula 1 wherein X is hydrogen, acetyl, or succinyl. Also preferred are those formula 1 compounds wherein
- A₁: is 
-His-Asn-Asp-Gly-Asp-,
-Asn-Asp-Gly-Asp-,
-Asp-Gly-Asp-,
-Gly-Asp-,
-Asp-, or a bond.
- A₂: is preferably Phe, β-(2- or 3-thienyl)alanine, Tyr, Trp, or pClPhe,;
- A₃,: Glu;
- A₅,: Ile;
- A₆,: Pro, Sar, DAla, Hyp or NMePgl;
- A₈,: Glu or Asp;
- A₉,: Tyr or a dipeptide fragment wherein at least one residue is Tyr or Tyr(SO₃H);
- A₁₀,: Leu, Asp, Asp-Glu, Leu-Gln, or Leu-Pro;
- Alk₁ and Alk₂,: each a methylene group;
- Y,: OH or NH₂; and
- B,: -S-S-.

Especially preferred are those peptide derivatives of formula 1 wherein either X is acetyl and A₁ is Gly-Asp or Asp or X is succinyl and A₁ is a bond and wherein
- A₂,: is Phe, β-(2-thienyl)alanine or Tyr;
- A₃,: Glu;
- A₅,: Ile;
- A₆,: Pro;
- A₈,: Glu or Asp;
- A₉,: Tyr or Ala-Tyr;
- A₁₀,: Gln, Asp, Leu-Pro, a bond, -Leu-Gln- or -Asp-Glu;
- R, R', R₁, and R₁',: each hydrogen;
- Alk₁ and Alk₂,: each a methylene group;
- B,: -S-S-; and
- Y,: OH.

The peptides of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide sythesizer. In this procedure an α-amino protected amino acid is bound to a resin support. The resin support employed can be any suitable resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid.

An example of a hydroxymethyl resin is described by Bodanszky et al., Chem. Ind. (London) 38, 1597-98 (1966). A chloromethylated resin is commercially available from Bio Rad Laboratories, Richmond, California, and the preparation of such a resin is described by Steward et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature of between 0°C and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and α-chlorobutyryl; (2) aromatic urethan type protecting groups such as benzyloxycarbonyl and substituted benzyloxycarbonyl, such as p-chlorobenzyloxycarbonyl, p-nitrobenzyloxycarbonyl, p-bromorbenzyloxycarbonyl, p-methoxybenzyloxycarbonyl,1-(p-biphenylyl)-1-methylethoxycarbonyl, α, α-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thio urethan type protecting groups such as phenylthiocarbonyl; (6) alkyl type protecting groups such as triphenylmethyl (trityl) and benzyl; and (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asn or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethyl-N'-(γ-dimethylaminopropylcarbodiimide); (2) cyanamides (e.g., N,N-dibenzylcyanamide); (3) ketenimines; (4) isoxazolium salts (e.g., N-ethyl-5-phenyl-isoxazolium-3'-sulfonate; (5) monocyclic nitrogen containing heterocyclic amides of aromatic character containing one through four nitrogens in the ring such as imidazolides, pyrazolides, and 1,2,4-triazolides. Specific heterocyclic amides that are useful include N,N'-carbonyldiimidazole and N,N-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Ala-O-Ala-Boc) and (8) nitrogen containing heterocyclic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp. 1-27 (1970). Applicants prefer the use of the symmetrical anhydride as a coupling reagent for all amino acids except Arg, Asn and Gln.

Each protected amino acid or amino acid sequence is introduced into the solid phase reactor in about a four-fold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl, said substituent being selected from halo (e.g., chloro, bromo, fluoro) and nitro (e.g., 2-chlorobenzyloxycarbonyl, p-nitrobenzyloxycarbonyl, 3,4-dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups can be removed by procedures well known in the art. Typically protecting group removal is done after the peptide chain synthesis is complete but the protecting groups can be removed at any other appropriate time.

In general, the cyclized peptides are prepared from an appropriate linear derivative either prior to or after removal of the linear peptide from the solid support. The compounds of structure 1 wherein B is a -S-S- group are prepared from the corresponding free sulfhydryl-containing, linear peptides by well known oxidative coupling technics such as by oxidizing the linear peptide with potassium ferricyanide described in, for example, Stewart et al., "Solid Phase Peptide Synthesis" (Freeman & Co., San Francisco 1969), Chapter 1, p. 95. The compounds of Structure 1 wherein B is a -S-Alk₃-S- group and Alk₃ is a (C₁-C₈)ethylene group can be prepared from the free sulfhydryl-containing linear peptides by reaction with a 1,2-dibromo derivative of an appropriate acyclic or cyclic, saturated or unsaturated alkyl in a manner analogous to that described in H. I. Mosberg and J. R. Omnaas, J. Amer. Chem. Soc. 107, 2986-2987 (1985). The compounds of structure 1 wherein B is a -S-Alk₃-S- group and Alk₃ is a (C₁-C₈)methylene group are prepared by reaction of the free sulfhydryl-containing linear peptide with an appropriate acyclic or cyclic, saturated or unsaturated alkyl ketone or aldehyde in a manner analogous to that described in J. Amer. Chem. Soc. 76, 1945 (1954). The preparation of those compounds of structure 1 wherein B is an -S- group can be accomplished in the manner set forth in K. Jost, Collect. Czech. Chem. Commun. 36, 218 (1971) and in United states Patent Number 4161521.

The anticoagulant dose of a peptide derivative of this invention is from 0.2 mg/kg to 250 mg/kg of patient body weight per day depending on the patient, the severity of the thromobotic condition to be treated and the peptide derivative selected. The suitable dose for a particular patient can be readily determined. Preferably from 1 to 4 daily doses would be administered typically with from 5 mg to 100 mg of active compound per dose.

Anticoagulant therapy is indicated for the treatment and prevention of a variety of thrombotic conditions, particularly coronary artery and cerebrovascular disease. Those experienced in this field are readily aware of the circumstances requiring anticoagulant therapy. The term "patient" used herein is taken to mean mammals such as primates, including humans, sheep, horses, cattle, pigs, dogs, cats, rats and mice.

Although some of the peptide derivatives may survive passage through the gut following oral administration, applicants prefer non-oral administration, for example, subcutaneous, intravenous, intramuscular or intraperitoneal; administration by depot injection; by implant preparation; or by application to the mucous membranes, such as, that of the nose, throat and bronchial tubes, for example, in an aerosol can containing a peptide derivative of this invention in a spray or dry powder form.

For parentral administration the compounds may be administered as injectable dosages of a solution or suspension of the compound in a physiologically acceptable diluent with a pharmaceutical carrier which can be a sterile liquid such as water and oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, a silicone rubber manufactured by the Dow-Corning Corporation.

This invention is illustrated by the following, nonlimiting examples.

### EXAMPLE 1

### Preparation of

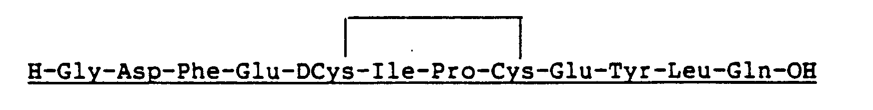

The peptide was synthesized by solid-phase methods using 0.1 mmol of a 0.66 mmol/g Boc-Gln-PAM resin. Double symmetrical anhydride couplings were performed with 2.0 mmol Na-Boc-amino acid (Peptides International) except in the case of Boc-Gln, which was coupled by the DCC/HOBT method. The side chain protection utilized was: Asp(Chx), Cys(pMeBzl), Glu(Bzl), Tyr(2-BrZ). Upon completion of the synthesis the Nα-Boc protection was removed with 50% trifluoroacetic acid in methylene chloride. The resin was washed three times with methylene chloride, neutralized with three washings of 10% diisopropylethylamine in methylene chloride, washed three times with methylene chloride, acetylated with N-acetylimidazole in methylene chloride, washed three times with methylene chloride, and dried in vacuo. The peptide was deprotected and cleaved from the resin with water and a small amount of 30% aqueous acetic acid. The extract was diluted to 2 l in volume with water and the pH adjusted to 8.5 with ammonium hydroxide. Potassium ferricyanide (0.01 N) was added to the solution until a yellow color persisted. The solution was stirred for 30 minutes, then the pH was adjusted to between 4 and 5 with acetic acid. The mixture was then stirred with Bio-Rad® AG3-X4A ion exchange resin for 2 hours. The mixture was filtered and the filtrate lyophilized.

The peptide was purified by desalting on a 92 x 2.6 cm Sephadex® G-15 column in 5% aqueous acetic acid and lyophilized. Preparative HPLC was performed on a C¹⁸ Vydac® 218TP1010 (250 x 10 mm) column with 24% acetonitrile in 0.1% aqueous trifluoroactic acid at 5 ml/min. The major peak was collected and lyophilized leaving 24 mg of the desired product and the dimer was isolated as a later eluting peak (14.2 mg). Homogeneity was determined by HPLC and TLC. HPLC Vydac® 218TP54 (250 x 4.6 mm) C¹⁸ column, 2 ml/min, t₀ = 1.8 min: time of elution with a 25-50% acetonitrile in 0.1% trifluoroacetic acid linear gradient at 1%/min. (HPLC) is 9.3 min.
FAB-MS: (M + H) = 1414 ± 1 mu (calcd. 1413). Amino acid analysis: (6N HCl hydrolysis; 24 hr. at 106°C) is shown in Table 1. 62% peptide content by weight.

In the same manner, the peptides of the following examples 2-4 were prepared.

### EXAMPLE 2

(H-Gly-Asp-Phe-Glu-DCys-Ile-Pro-Cys-Glu-Tyr-Leu-Gln-OH)2
|                |
S                S
|                |

### EXAMPLE 3

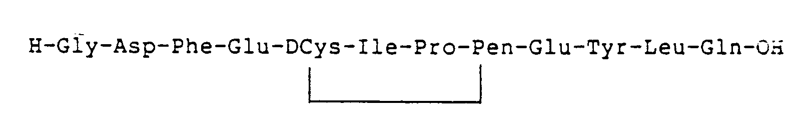
H-Gly-Asp-Phe-Glu-DCys-Ile-Pro-Pen-Glu-Tyr-Leu-Gln-OH

### EXAMPLE 4

(H-Gly-Asp-Phe-Glu-DCys-Ile-Pro-Pen-Glu-Tyr-Leu-Gln-OH)₂
|                |
S                S
|                |

The peptides of examples 1 - 4 have the following properties:

| EXAMPLE NO. | Amino Acids Analysis (6N HCl Hydrolysis; 24 Hrs at 106°C) | | | | | | | |
|---|---|---|---|---|---|---|---|---|
| | Asx | Glx | Pro | Gly | Ile* | Leu | Tyr | Phe |
| 1 | 1.03(1) | 3.08(3) | 0.91(1) | 1.01(1) | 0.72(1) | 0.98(1) | 0.95(1) | 1.04(1) |
| 2 | 1.00(1) | 3.10(3) | 0.97(1) | 0.99(1) | 0.91(1) | 1.01(1) | 0.88(1) | 0.94(1) |
| 3 | 1.00(1) | 3.00(3) | 0.83(1) | 1.01(1) | 0.68(1) | 1.01(1) | 1.00(1) | 0.99(1) |
| 4 | 1.01(1) | 2.94(3) | 0.98(1) | 1.02(1) | 0.85(1) | 1.02(1) | 1.02(1) | 1.01(1) |

| | | | | | | | | |
|---|---|---|---|---|---|---|---|---|
| * Portion converted to allo-Ile during hydrolysis not quantitated. | | | | | | | | |

| Physical Characteristics | | |
|---|---|---|
| EXAMPLE NO. | HPLC tᵣ (min) | FAB-MS (M + H) |
| 1 | 9.3 | 1414 |
| 2 | 12.8 | 2826 |
| 3 | 9.2 | 1445 |
| 4 | 13.6 | 2883 |

## Claims (Claims for the following Contracting State(s): AT, BE, CH, DE, FR, GB, IT, LI, LU, NL, SE)

1. A peptide derivative of the formula 1 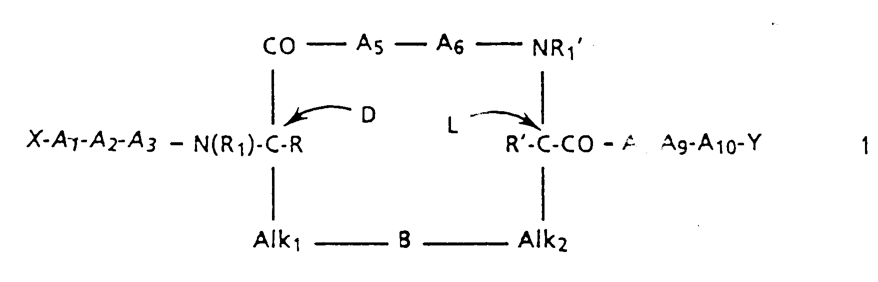 wherein
X is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl;
A₁ is a bond or is a peptide containing from 1 to 5 residues of any amino acid;
A₂ is Phe, SubPhe mono- or di-substituted phenylalanine, β-(2- and 3-thienyl)alanine, β-(2- and 3-furanyl)alanine, β-(2-, 3-, and 4-pyridyl)alanine, β-(benzothienyl-2- and 3-yl)alanine, β-(1- and 2-naphthyl)alanine, Tyr, or Trp;
A₃ is Glu or Asp;
A₅ is Ile, Val, Leu, Nle, or Thr;
A₆ is Pro, Hyp, 3,4-dehydroPro, thiazolidine-4-carboxylate, Sar, NMePgl (N-methyl-phenylglycine), or any amino acids having a D-configuration;
A₈ is Glu or Asp;
A₉ is a lipophilic amino acid selected from Tyr, Tyr(SO₃H), Trp, Phe, Leu, Nle, Ile, Val, His, and Pro or is a dipeptide containing at least one of these lipophilic amino acids;
A₁₀ is a bond or is a peptide fragment containing from one to five residues of any amino acid;
Y is a carboxy terminal residue selected from OH, (C₁-C₆)alkoxy, amino, mono- or di-(C₁-C₄)alkyl substituted amino, or benzylamino;
R, R', R₁, and R₁' are each selected from a hydrogen or (C₁-C₄)alkyl group;
B is selected from -S-, -S-S-, or -S-Alk₃-S-;
Alk₁, Alk₂, and Alk₃ are each selected from a (C₁-C₈)methylene or ethylene group;
and wherein "D" and "L" indicate that the stereochemistry of the indicated carbon is that corresponding to D-cysteine and L-cysteine, respectively, as well as a dimer or a mixture of a peptide derivative and the dimer thereof and their pharmaceutically acceptable salts.

2. A peptide derivative of claim 1 wherein A₂ is Phe, β-(2-or 3-thienyl)alanine, or Tyr.

3. A peptide derivative of anyone of claims 1 or 2 wherein A₃ is Glu.

4. A peptide derivative of anyone of claims 1 to 3 wherein A₅ is Ile.

5. A peptide derivative of anyone of claims 1 to 4 wherein A₆ is Pro.

6. A peptide derivative of anyone of claims 1 to 5 wherein A₈ is Glu or Asp.

7. A peptide derivative of anyone of claims 1 to 6 wherein A₉ is Tyr, Ala-Tyr, Ala-Tyr(SO₃H) or Glu-Leu.

8. A peptide derivative of anyone of claims 1 to 7 wherein A₁₀ is Gln, Leu, Asp, Asp-Glu, Leu-Pro, Leu-Gln, Leu-Ala, or a bond.

9. The peptide derivative of anyone of claims 1 to 8 wherein X is H, acetyl, or succinyl.

10. The peptide derivative of anyone of claims 1 to 9 wherein Y is OH or NH₂.

11. The peptide derivative of anyone of claims 1 to 10 wherein R, R', R₁, R₁' are each a hydrogen.

12. The peptide derivative of anyone of claims 1 to 11 wherein B is the group -S-S-.

13. The peptide derivative of anyone of claims 1 to 12 wherein Alk₁ and Alk₂ are each a methylene group of the formula -(CH₂)-.

14. The peptide derivative of anyone of claims 1 to 13 wherein A₁ is

His-Asn-Asp-Gly-Asp,
Asn-Asp-Gly-Asp,
Asp-Gly-Asp,
Gly-Asp,
Asp, or
a bond.

15. A process for the preparation of a peptide derivative of anyone of claims 1 to 14, of a dimer thereof, or of a mixture of the dimer and said polypeptide comprising preparing the free sulfhydryl-containing linear peptide by solid phase sequential or block synthesis, gene cloning, or a combination thereof and subsequently subjecting the linear peptide to an oxidative coupling.

16. A solid phase sequential or block synthesis process for the preparation of a peptide derivative of anyone of claims 1 to 14, of a dimer thereof, or of a mixture of the dimer and said polypeptide comprising binding a suitably protected amino acid of formula A₁ having the meaning as given in the preceding claims to an activated resin support, subsequently binding the other alpha amino protected amino acids of the formula A₂ to A₁₀ having the meaning as given in the preceding claims to the terminal amino group of the growing peptidic chain which has meanwhile been exposed by removing its amino protecting group, and finally subjecting the linear peptide to an oxidative coupling.

17. A peptide derivative of anyone of claims 1 to 14 or a dimer thereof, or a mixture of these compounds for use as pharmaceutically active substance.

18. A peptide derivative of anyone of claims 1 to 14 or a dimer thereof, or a mixture of these compounds for use as an anticoagulant.

19. A pharmaceutical composition containing an anticoagulant effective amount of a peptide derivative of anyone of claims 1 to 14, of a dimer thereof, or of a mixture of these compounds, and optionally a pharmaceutically acceptable carrier and/or diluent.

20. A peptide derivative of claim 1 which is

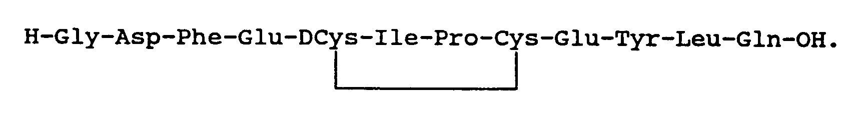
H-Gly-Asp-Phe-Glu-DCys-Ile-Pro-Cys-Glu-Tyr-Leu-Gln-OH.


21. A peptide derivative of claim 1 which is 
(H-Gly-Asp-Phe-Glu-DCys-Ile-Pro-Cys-Glu-Tyr-Leu-Gln-OH)2,
S S
 wherein the depicted bonds occur between the dimer peptide.

22. A peptide derivative of claim 1 which is

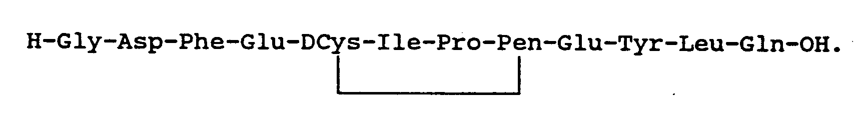
H-Gly-Asp-Phe-Glu-DCys-Ile-Pro-Pen-Glu-Tyr-Leu-Gln-OH.

23. A peptide derivative of claim 1 which is 
(H-Gly-Asp-Phe-Glu-DCys-Ile-Pro-Pen-Glu-Tyr-Leu-Gln-OH)2,
S
S
 wherein the depicted bonds occur between the dimer peptide.

## Claims (Claims for the following Contracting State(s): ES)

1. A process for preparing a peptide derivative of the formula 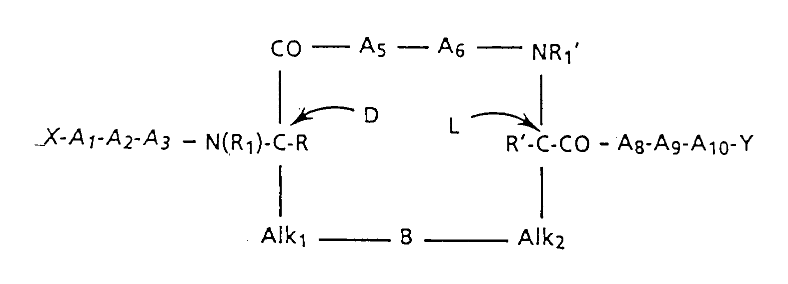 wherein
X is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl;
A₁ is a bond or is a peptide containing from 1 to 5 residues of any amino acid;
A₂ is Phe, SubPhe mono- or di-substituted phenylalanine, β-(2- and 3-thienyl)alanine, β-(2- and 3-furanyl)alanine, β-(2-, 3-, and 4-pyridyl)alanine, β-(benzothienyl-2- and 3-yl)alanine, β-(1- and 2-naphthyl)alanine, Tyr, or Trp;
A₃ is Glu or Asp;
A₅ is Ile, Val, Leu, Nle, or Thr;
A₆ is Pro, Hyp, 3,4,-dehydroPro, thiazolidine-4-carboxylate, Sar, NMePgl (N-methyl-phenylglycine), or any amino acids having a D-configuration;
A₈ is Glu or Asp;
A₉ is a lipophilic amino acid selected from Tyr, Tyr(SO₃H), Trp, Phe, Leu, Nle, Ile, Val, His, and Pro or is a dipeptide containing at least one of these lipophilic amino acids;
A₁₀ is a bond or is a peptide fragment containing from one to five residues of any amino acid;
Y is a carboxy terminal residue selected from OH, (C₁-C₆)alkoxy, amino, mono- or di-(C₁-C₄)alkyl substituted amino, or benzylamino;
R, R', R₁, and R₁' are each selected from a hydrogen or (C₁-C₄)alkyl group;
B is selected from -S-, -S-S-, or -S-Alk₃S-;
Alk₁, Alk₂, and Alk₃ are each selected from a (C₁-C₈)methylene or ethylene group;
and wherein "D" and "L" indicate that the stereochemistry of the indicated carbon is that corresponding to D-cysteine and L-cysteine, respectively, as well as a dimer or a mixture of a peptide derivative and the dimer thereof comprising preparing the free sulfhydryl-containing linear peptide by solid phase sequential or block synthesis, gene cloning, or a combination thereof and subsequently subjecting the linear peptide to an oxidative coupling.

2. A solid phase sequential or block synthesis process for preparing a peptide derivative of the formula 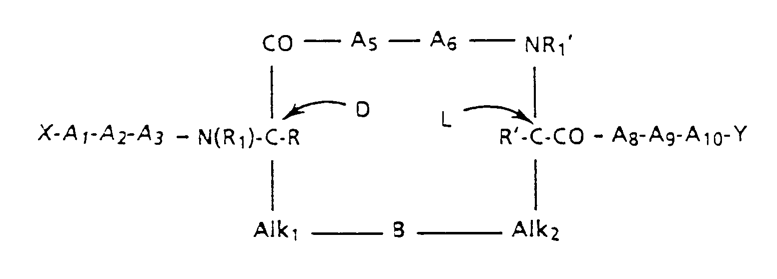 wherein
X is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl;
A₁ is a bond or is peptide containing from 1 to 5 residues of any amino acid;

3. A process of claim 1 or 2 wherein A₂ is Phe, β-(2- or 3-thienyl)alanine, or Tyr.

4. A process according to anyone of claims 1 to 3 wherein A₃ is Glu.

5. A process according to anyone of claims 1 to 4 wherein A₅ is Ile.

6. A process according to anyone of claims 1 to 5 wherein A₆ is Pro.

7. A process according to anyone of claims 1 to 6 wherein A₈ is Glu or Asp.

8. A process according to anyone of claims 1 to 7 wherein A₉ is Tyr, Ala-Tyr, Ala-Tyr(SO₃H) or Glu-Leu.

9. A process according to anyone of claims 1 to 8 wherein A₁₀ is Gln, Leu, Asp, Asp-Glu, Leu-Pro, Leu-Gln, Leu-Ala, or a bond.

10. A process according to anyone of claims 1 to 9 wherein X is H, acetyl, or succinyl.

11. A process according to anyone of claims 1 to 10 wherein Y is OH or NH₂.

12. A process according to anyone of claims 1 to 11 wherein R, R', R₁, R₁' are each hydrogen.

13. A process according to anyone of claims 1 to 12 wherein B is the group -S-S-.

14. A process according to anyone of claims 1 to 13 wherein Alk₁ and Alk₂ are each a methylene group of the formula -(CH₂)-.

15. A process according to anyone of claims 1 to 14 wherein A₁ is

His-Asn-Asp-Gly-Asp,
Asn-Asp-Gly-Asp,
Asp-Gly-Asp,
Gly-Asp,
Asp, or
a bond.

16. Use of a compound obtainable according to the process of anyone of claims 1 to 15 for the preparation of a pharmaceutical composition.

17. Use of a compound obtainable according to the process of anyone of claims 1 to 15 for the preparation of an anticoagulant.

18. A process according to claim 1 for preparing a peptide derivative which is

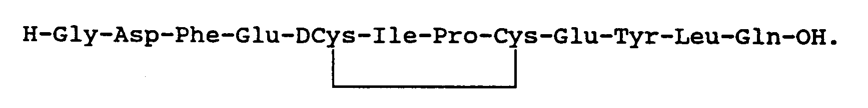
H-Gly-Asp-Phe-Glu-DCys-Ile-Pro-Cys-Glu-Tyr-Leu-Gln-OH.


19. A process according to claim 1 for preparing a peptide derivative which is 
(H-Gly-Asp-Phe-Glu-DCys-Ile-Pro-Cys-Glu-Tyr-Leu-Gln-OH)₂,
S S
 wherein the depicted bonds occur between the dimer peptide.

20. A process according to claim 1 for preparing a peptide derivative which is

H-Gly-Asp-Phe-Glu-DCys-Ile-Pro-Pen-Glu-Tyr-Leu-Gln-OH.

21. A process according to claim 1 for preparing a peptide derivative which is 
(H-Gly-Asp-Phe-Glu-DCys-Ile-Pro-Pen-Glu-Tyr-Leu-Gln-OH)2,
S S
 wherein the depicted bonds occur between the dimer peptide.

## Claims (Claims for the following Contracting State(s): GR)

1. A peptide derivative of the formula 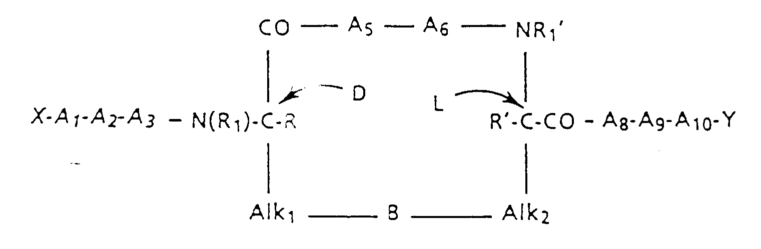 wherein
X is an amino terminal residue selected from hydrogen, one or two alkyl groups of from 1 to 6 carbon atoms, one or two acyl groups of from 2 to 10 carbon atoms, carbobenzyloxy, or t-butyloxycarbonyl;
A₁ is a bond or is a peptide containing from 1 to 5 residues of any amino acid;
A₂ is Phe, SubPhe mono- or di-substituted phenylalanine, β-(2- and 3-thienyl)alanine, β-(2- and 3-furanyl)alanine, β-(2-, 3-, and 4-pyridyl)alanine, β-(benzothienyl-2- and 3-yl)alanine, β-(1- and 2-naphthyl)alanine, Tyr, or Trp;
A₃ is Glu or Asp;
A₅ is Ile, Val, Leu, Nle, or Thr;
A₆ is Pro, Hyp, 3,4-dehydroPro, thiazolidine-4-carboxylate, Sar, NMePgl (N-methyl-phenylglycine), or any amino acids having a D-configuration;
A₈ is Glu or Asp;
A₉ is a lipophilic amino acid selected from Tyr, Tyr(SO₃H), Trp, Phe, Leu, Nle, Ile, Val, His, and Pro or is a dipeptide containing at least one of these lipophilic amino acids;
A₁₀ is a bond or is a peptide fragment containing from one to five residues of any amino acid;
Y is a carboxy terminal residue selected from OH, (C₁-C₆)alkoxy, amino, mono- or di-(C₁-C₄)alkyl substituted amino, or benzylamino;
R, R', R₁, and R₁' are each selected from a hydrogen or (C₁-C₄)alkyl group;
B is selected from -S-, -S-S -, or -S-Alk₃-S-;
Alk₁, Alk₂, and Alk₃ are each selected from a (C₁-C₃)methylene] or ethylene group;
and wherein "D" and "L" indicate that the stereochemistry of the indicate carbon is that corresponding to D-cysteine and L-cysteine, respectively, as well as a diner or a mixture of a peptide derivative and the dimer thereof and their pharmaceutically acceptable salts.

2. A peptide derivative of claim 1 wherein A₂ is Phe, β-(2- or 3-thienyl)alanine, or Tyr.

3. A peptide derivative of anyone of claims 1 or 2 wherein A₃ is Glu.

4. A peptide derivative of anyone of claims 1 to 3 wherein A₅ is Ile.

5. A peptide derivative of anyone of claims 1 to 4 wherein A₆ is Pro.

6. A peptide derivative of anyone of claims 1 to 5 wherein A₈ is Glu or Asp.

7. A peptide derivative of anyone of claims 1 to 6 wherein A₉ is Tyr, Ala-Tyr, Ala-Tyr(SO₃H) or Glu-Leu.

8. A peptide derivative of anyone of claims 1 to 7 wherein A₁₀ is Gln, Leu, Asp, Asp-Glu, Leu-Pro, Leu-Gln, Leu-Ala, or a bond.

9. The peptide derivative of anyone of claims 1 to 8 wherein X is H, acetyl, or succinyl.

10. The peptide derivative of anyone of claims 1 to 9 wherein Y is OH or NH₂.

11. The peptide derivative of anyone of claims 1 to 10 wherein R, R', R₁, R₁' are each a hydrogen.

12. The peptide derivative of anyone of claims 1 to 11 wherein B is the group -S-S-.

13. The peptide derivative of anyone of claims 1 to 12 wherein Alk₁ and Alk₂ are each a methylene group of the formula -(CH₂)-.

14. The peptide derivative of anyone of claims 1 to 13 wherein A₁ is

His-Asn-Asp-Gly-Asp,
Asn-Asp-Gly-Asp,
Asp-Gly-Asp,
Gly-Asp,
Asp, or
a bond.

15. A process for the preparation of a peptide derivative of anyone of claims 1 to 14, of a dimer thereof, or of a mixture of the dimer and said polypeptide comprising preparing the free sulfhydryl-containing linear peptide by solid phase sequential or block synthesis, gene cloning, or a combination thereof and subsequently subjecting the linear peptide to an oxidative coupling.

16. A solid phase sequential or block synthesis process for the preparation of a peptide derivative of anyone of claims 1 to 14, of a dimer thereof, or of a mixture of the dimer and said polypeptide comprising binding a suitably protected amino acid of formula A₁ having the meaning as given in the preceding claims to an activated resin support, subsequently binding the other alpha amino protected amino acids of the formula A₂ to A₁₀ having the meaning as given in the preceding claims to the terminal amino group of the growing peptidic chain which has meanwhile been exposed by removing its amino protecting group, and finally subjecting the linear peptide to an oxidative coupling.

17. A peptide derivative of anyone of claims 1 to 14 or a dimer thereof, or a mixture of these compounds for use as pharmaceutically active compounds.

18. A peptide derivative or anyone of claims 1 to 14 or a dimer thereof, or a mixture of these compounds for use as an anticoagulant.

19. A peptide derivative of claim 1 which is

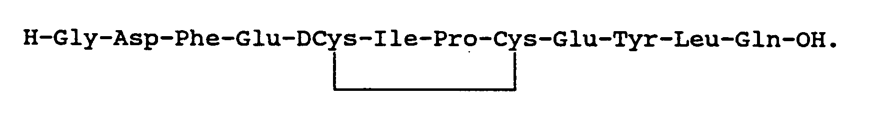
H-Gly-Asp-Phe-Glu-DCys-Ile-Pro-Cys-Glu-Tyr-Leu-Gln-OH.

20. A peptide derivative of claim 1 which is 
(H-Gly-Asp-Phe-Glu-DCys-Ile-Pro-Cys-Glu-Tyr-Leu-Gln-OH)2,
S
S
 wherein the depicted bonds occur between the dimer peptide.

21. A peptide derivative of claim 1 which is

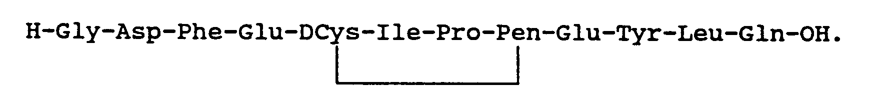
H-Gly-Asp-Phe-Glu-DCys-Ile-Pro-Pen-Glu-Tyr-Leu-Gln-OH.

22. A peptide derivative of claim 1 which is 
(H-Gly-Asp-Phe-Glu-DCys-Ile-Pro-Pen-Glu-Tyr-Leu-Gln-OH)2,
S
S
 wherein the depicted bonds occur between the dimer peptide.

## Patentansprüche (Patentansprüche für folgende(n) Vertragsstaat(en): AT, BE, CH, DE, FR, GB, IT, LU, NL, SE)

1. Peptid-Derivat der Formel 1 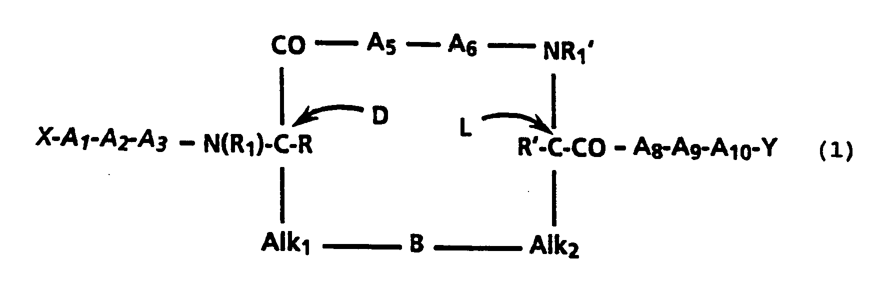 in der
X einen aminoterminalen Rest, ausgewählt aus Wasserstoff, einem oder zwei Alkylresten mit 1 bis 6 Kohlenstoffatomen, einem oder zwei Acylresten mit 2 bis 10 Kohlenstoffatomen, Carbobenzyloxy- oder ter.-Butyloxycarbonylgruppen bedeutet;
A₁ eine Bindung ist, oder ein Peptid mit 1 bis 5 Resten irgendeiner Aminosäure darstellt;
A₂ eine der Gruppen Phe, SubPhe (mono- oder disubstituiertes Phenylalanin), β-(2- und 3-Thienyl)-alanin, β-(2- und 3-Furanyl)-alanin, β-(2-, 3-, und 4-Pyridyl)-alanin, β-(Benzothienyl-2- und 3-yl)-alanin, β-(1- und 2-Naphthyl)-alanin, Tyr oder Trp bedeutet;
A₃ Glu oder Asp ist;
A₅ Ile, Val, Leu, Nle oder Thr bedeutet;
A₆ eine der Gruppen Pro, Hyp, 3,4-DehydroPro, Thiazolidin-4-carboxylat, Sar, NMePgl (N-Methylphenylglycin) oder irgendeine Aminosäure mit D-Konfiguration darstellt;
A₈ Glu oder Asp ist;
A₉ eine lipophile Aminosäure, ausgewählt aus Tyr, Tyr(SO₃H), Trp, Phe, Leu, Nle, Ile, Val, His und Pro bedeutet oder ein mindestens eine dieser lipophilen Aminosäuren enthaltendes Dipeptid ist;
A₁₀ eine Bindung ist oder ein Peptidfragment mit 1 bis 5 Resten irgendwelcher Aminosäuren darstellt;
Y einen carboxyterminalen Rest, ausgewählt aus OH, (C₁-C₆)-Alkoxy-, Amino-, Mono- oder Di-(C₁-C₄)alkylsubstituierten Amino- oder Benzylaminogruppen bedeutet;
R, R', R₁ und R₁' jeweils ausgewählt sind aus Wasserstoffatomen oder (C₁-C₄)-Alkylresten;
B ausgewählt ist aus -S-, -S-S- oder -S-Alk₃-S-;
Alk₁, Alk₂ und Alk₃ jeweils ausgewählt sind aus (C₁-C₈)-Methylen- oder Ethylengruppen;
und wobei "D" und "L" anzeigen, daß die Stereochemie am angegebenen Kohlenstoffatom diejenige ist, die dem D-Cystein bzw. L-Cystein entspricht, sowie ein Dimer oder ein Gemisch eines Peptidderivates und des Dimers davon, und ihre pharmazeutisch verträglichen Salze.

2. Peptidderivat nach Anspruch 1, wobei A₂ Phe, β(2 oder 3-Thienyl)-alanin oder Tyr bedeutet.

3. Peptidderivat nach einem der Ansprüche 1 oder 2, wobei A₃ Glu ist.

4. Peptidderivat nach einem der Ansprüche 1 bis 3, wobei A₅ Ile ist.

5. Peptidderivat nach einem der Ansprüche 1 bis 4, wobei A₆ Pro ist.

6. Peptidderivat nach einem der Ansprüche 1 bis 5, wobei A₈ Glu oder Asp ist.

7. Peptidderivat nach einem der Ansprüche 1 bis 6, wobei A₉ Tyr, Ala-Tyr, Ala-Tyr(SO₃H) oder Glu-Leu ist.

8. Peptidderivat nach einem der Ansprüche 1 bis 7, wobei A₁₀ Gln, Leu, Asp, Asp-Glu, Leu-Pro, Leu-Gln, Leu-Ala oder eine Bindung bedeutet.

9. Peptidderivat nach einem der Ansprüche 1 bis 8, wobei X ein Wasserstoffatom, eine Acetyl- oder Succinylgruppe bedeutet.

10. Peptidderivat nach einem der Ansprüche 1 bis 9, wobei Y OH oder NH₂ ist.

11. Peptidderivat nach einem der Ansprüche 1 bis 10, wobei R, R', R₁ und R₁' jeweils ein Wasserstoffatom sind.

12. Peptidderivat nach einem der Ansprüche 1 bis 11, wobei B die Gruppe -S-S- bedeutet.

13. Peptidderivat nach einem der Ansprüche 1 bis 12, wobei Alk₁ und Alk₂ jeweils eine Methylengruppe der Formel -(CH₂)- sind.

14. Peptidderivat nach einem der Ansprüche 1 bis 13, wobei A₁ eine der Gruppen 
His-Asn-Asp-Gly-Asp,
Asn-Asp-Gly-Asp,
Asp-Gly-Asp,
Gly-Asp,
Asp
 oder eine Bindung ist.

15. Verfahren zur Herstellung eines Peptidderivates nach einem der Ansprüche 1 bis 14, eines Dimers davon oder eines Gemisches des Dimeren und des Polypeptids, umfassend die Herstellung des freien sulfhydrylhaltigen linearen Peptids durch Festphasen-Sequenz- oder Blocksynthese, Genclonierung oder eine Kombination davon und anschließend die Durchführung von oxidativer Kupplung mit dem linearen Peptid.

16. Festphasen-Sequenz- oder Blocksyntheseverfahren zur Herstellung eines Peptidderivates nach einem der Ansprüche 1 bis 14, oder eines Dimeren davon oder eines Gemisches des Dimeren und des Polypeptids, umfassend die Bindung einer in geeigneter Weise geschützten Aminosäure der Formel A₁, die die in den vorstehenden Ansprüchen angegebene Bedeutung hat, an einen aktivierten Harzträger, anschließend Bindung der anderen, an der α-Aminogruppe geschützten Aminosäuren der Formeln A₂ bis A₁₀, die die in den vorangehenden Ansprüchen angegebenen Bedeutungen haben, an die endständige Aminogruppe der wachsenden Peptidkette, die in der Zwischenzeit durch Entfernung ihrer Aminoschutzgruppe freigesetzt worden ist, und schließlich Durchführung einer oxidativen Kupplung mit dem linearen Peptid.

17. Peptidderivat nach einem der Ansprüche 1 bis 14 oder ein Dimer davon oder ein Gemisch dieser Verbindungen zur Verwendung als pharmazeutischer Wirkstoff.

18. Peptidderivat nach einem der Ansprüche 1 bis 14 oder ein Dimer davon oder ein Gemisch dieser Verbindungen zur Verwendung als antikoagulierender Wirkstoff.

19. Arzneimittel, enthaltend eine antikoagulierend wirksame Menge eines Peptidderivates nach einem der Ansprüche 1 bis 14, eines Dimeren davon oder eines Gemisches dieser Verbindungen, und gegebenenfalls einen pharmazeutisch verträglichen Träger und/oder ein Verdünnungsmittel.

20. Peptid-Derivat nach Anspruch 1, nämlich

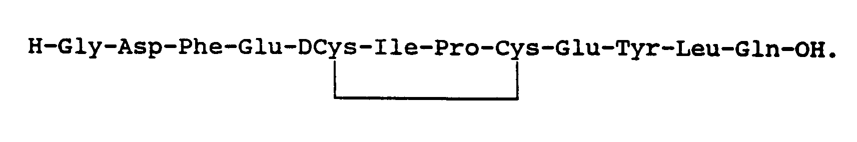
H-Gly-Asp-Phe-Glu-DCys-Ile-Pro-Cys-Glu-Tyr-Leu-Gln-OH.

21. Peptid-Derivat nach Anspruch 1, nämlich 
(H-Gly-Asp-Phe-Glu-DCys-Ile-Pro-Cys-Glu-Tyr-Leu-Gln-OH)2
                    |           |
                    S           S
                    |           |
 wobei die gezeichneten Bindungen innerhalb des Dimer-Peptids bestehen.

22. Peptid-Derivat nach Anspruch 1, nämlich

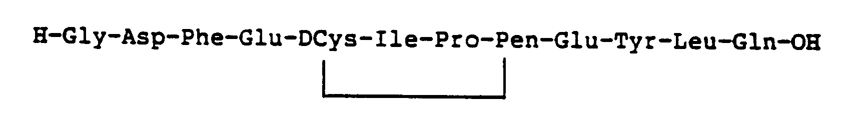
H-Gly-Asp-Phe-Glu-DCys-Ile-Pro-Pen-Glu-Tyr-Leu-Gln-OH


23. Peptid-Derivat nach Anspruch 1, nämlich 
(H-Gly-Asp-Phe-Glu-DCys-Ile-Pro-Pen-Glu-Tyr-Leu-Gln-OH)2
                    |           |
                    S           S
                    |           |
 wobei die gezeichneten Bindungen innerhalb des Dimer-Peptids bestehen.

## Patentansprüche (Patentansprüche für folgende(n) Vertragsstaat(en): ES)

1. Verfahren zur Herstellung eines Peptid-Derivates der Formel 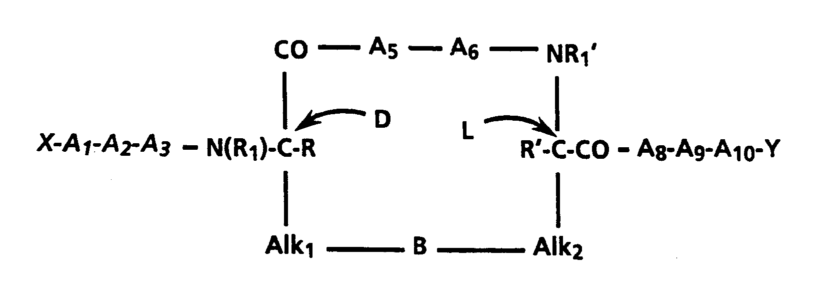 in der
X einen aminoterminalen Rest, ausgewählt aus Wasserstoff, einem oder zwei Alkylresten mit 1 bis 6 Kohlenstoffatomen, einem oder zwei Acylresten mit 2 bis 10 Kohlenstoffatomen, Carbobenzyloxy- oder ter.-Butyloxycarbonylgruppen bedeutet;
A₁ eine Bindung ist, oder ein Peptid mit 1 bis 5 Resten irgendeiner Aminosäure darstellt;
A₂ eine der Gruppen Phe, SubPhe (mono- oder disubstituiertes Phenylalanin), β-(2- und 3-Thienyl)-alanin, β-(2- und 3-Furanyl)-alanin, β-(2-, 3-, und 4-Pyridyl)-alanin, β-(Benzothienyl-2- und 3-yl)-alanin, β-(1- und 2-Naphthyl)-alanin, Tyr oder Trp bedeutet;
A₃ Glu oder Asp ist;
A₅ Ile, Val, Leu, Nle oder Thr bedeutet;
A₆ eine der Gruppen Pro, Hyp, 3,4-DehydroPro, Thiazolidin-4-carboxylat, Sar, NMePgl (N-Methylphenylglycin) oder irgendeine Aminosäure mit D-Konfiguration darstellt;
A₈ Glu oder Asp ist;
A₉ eine lipophile Aminosäure, ausgewählt aus Tyr, Tyr(SO₃H), Trp, Phe, Leu, Nle, Ile, Val, His und Pro bedeutet oder ein mindestens eine dieser lipophilen Aminosäuren enthaltendes Dipeptid ist;
A₁₀ eine Bindung ist oder ein Peptidfragment mit 1 bis 5 Resten irgendwelcher Aminosäuren darstellt;
Y einen carboxyterminalen Rest, ausgewählt aus OH, (C₁-C₆)-Alkoxy-, Amino-, Mono- oder Di-(C₁-C₄)alkylsubstituierten Amino- oder Benzylaminogruppen bedeutet;
R, R', R₁ und R₁' jeweils ausgewählt sind aus Wasserstoffatomen oder (C₁-C₄)-Alkylresten;
B ausgewählt ist aus -S-, -S-S- oder -S-Alk₃-S-;
Alk₁, Alk₂ und Alk₃ jeweils ausgewählt sind aus (C₁-C₈)-Methylen- oder Ethylengruppen;
und wobei "D" und "L" anzeigen, daß die Stereochemie am angegebenen Kohlenstoffatom diejenige ist, die dem D-Cystein bzw. L-Cystein entspricht, sowie ein Dimer oder ein Gemisch eines Peptidderivates und des Dimers davon, umfassend die Herstellung des freien sulfhydrylhaltigen linearen Peptids durch Festphasen-Sequenz- oder Blocksynthese, Genclonierung oder eine Kombination davon und anschließend die Durchführung von oxidativer Kupplung mit dem linearen Peptid.

2. Festphasen-Sequenz- oder Blocksyntheseverfahren zur Herstellung eines Peptidderivates der Formel 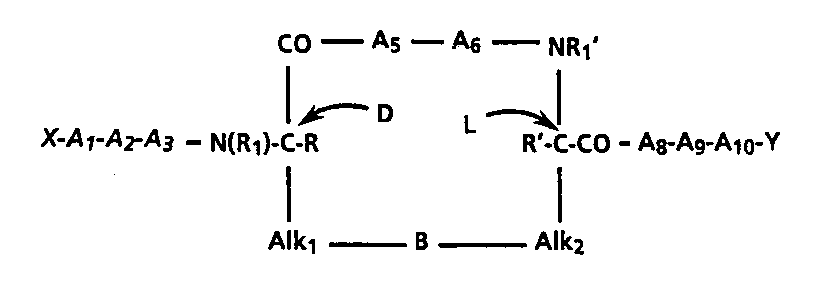 in der
X einen aminoterminalen Rest, ausgewählt aus Wasserstoff, einem oder zwei Alkylresten mit 1 bis 6 Kohlenstoffatomen, einem oder zwei Acylresten mit 2 bis 10 Kohlenstoffatomen, Carbobenzyloxy- oder ter.-Butyloxycarbonylgruppen bedeutet;
A₁ eine Bindung ist, oder ein Peptid mit 1 bis 5 Resten irgendeiner Aminosäure darstellt;
A₂ eine der Gruppen Phe, SubPhe, β-(2- und 3-Thienyl)-alanin, β-(2- und 3-Furanyl)-alanin, β-(2-, 3-, und 4-Pyridyl)-alanin, β-(Benzothienyl-2- und 3-yl)-alanin, β-(1- und 2-Naphthyl)-alanin, Tyr oder Trp bedeutet;
A₃ Glu oder Asp ist;
A₅ Ile, Val, Leu, Nle oder Thr bedeutet;
A₆ eine der Gruppen Pro, Hyp, 3,4-DehydroPro, Thiazolidin-4-carboxylat, Sar, NMePgl oder irgendeine Aminosäure mit D-Konfiguration darstellt;
A₈ Glu oder Asp ist;
A₉ eine lipophile Aminosäure, ausgewählt aus Tyr, Tyr(SO₃H), Trp, Phe, Leu, Nle, Ile, Val, His und Pro bedeutet oder ein mindestens eine dieser lipophilen Aminosäuren enthaltendes Dipeptid ist;
A₁₀ eine Bindung ist oder ein Peptidfragment mit 1 bis 5 Resten irgendwelcher Aminosäuren darstellt;
Y einen carboxyterminalen Rest, ausgewählt aus OH, (C₁-C₆)-Alkoxy-, Amino-, Mono- oder Di-(C₁-C₄)alkylsubstituierten Amino- oder Benzylaminogruppen bedeutet;
R, R', R₁ und R₁' jeweils ausgewählt sind aus Wasserstoffatomen oder (C₁-C₄)-Alkylresten;
B ausgewählt ist aus -S-, -S-S- oder -S-Alk₃-S-;
Alk₁, Alk₂ und Alk₃ jeweils ausgewählt sind aus (C₁-C₈)-Methylen- oder Ethylengruppen;
und wobei "D" und "L" anzeigen, daß die Stereochemie am angegebenen Kohlenstoffatom diejenige ist, die dem D-Cystein bzw. L-Cystein entspricht, sowie ein Dimer oder ein Gemisch eines Peptidderivates und des Dimers davon, umfassend die Bindung einer in geeigneter Weise geschützten Aminosäure der Formel A₁, an einen aktivierten Harzträger, anschließend Bindung der anderen, an der α-Aminogruppe geschützten Aminosäuren der Formeln A₂ bis A₁₀, an die endständige Aminogruppe der wachsenden Peptidkette, die in der Zwischenzeit durch Entfernung ihrer Aminoschutzgruppe freigesetzt worden ist, und schließlich Durchführung einer oxidativen Kupplung mit dem linearen Peptid.

3. Verfahren nach Anspruch 1 oder 2, wobei A₂ Phe, β(2 oder 3-Thienyl)-alanin oder Tyr bedeutet.

4. Verfahren nach einem der Ansprüche 1 bis 3, wobei A₃ Glu ist.

5. Verfahren nach einem der Ansprüche 1 bis 4, wobei A₅ Ile ist.

6. Verfahren nach einem der Ansprüche 1 bis 5, wobei A₆ Pro ist.

7. Verfahren nach einem der Ansprüche 1 bis 6, wobei A₈ Glu oder Asp ist.

8. Verfahren nach einem der Ansprüche 1 bis 7, wobei A₉ Tyr, Ala-Tyr, Ala-Tyr(SO₃H) oder Glu-Leu ist.

9. Verfahren nach einem der Ansprüche 1 bis 8, wobei A₁₀ Gln, Leu, Asp, Asp-Glu, Leu-Pro, Leu-Gln, Leu-Ala oder eine Bindung bedeutet.

10. Verfahren nach einem der Ansprüche 1 bis 9, wobei X ein Wasserstoffatom, eine Acetyl- oder Succinylgruppe bedeutet.

11. Verfahren nach einem der Ansprüche 1 bis 10, wobei Y OH oder NH₂ ist.

12. Verfahren nach einem der Ansprüche 1 bis 11, wobei R, R', R₁ und R₁' jeweils ein Wasserstoffatom sind.

13. Verfahren nach einem der Ansprüche 1 bis 12, wobei B die Gruppe -S-S- bedeutet.

14. Verfahren nach einem der Ansprüche 1 bis 13, wobei Alk₁ und Alk₂ jeweils eine Methylengruppe der Formel -(CH₂)-sind.

15. Verfahren nach einem der Ansprüche 1 bis 14, wobei A₁ eine der Gruppen 
His-Asn-Asp-Gly-Asp,
Asn-Asp-Gly-Asp,
Asp-Gly-Asp,
Gly-Asp,
Asp
 oder eine Bindung ist.

16. Verwendung einer nach dem Verfahren eines der Ansprüche 1 bis 15 erhältlichen Verbindung zur Herstellung eines Arzneimittels.

17. Verwendung einer nach dem Verfahren eines der Ansprüche 1 bis 15 erhältlichen Verbindung zur Herstellung eines Gerinnungshemmers.

18. Verfahren zur Herstellung eines Peptid-Derivates nach Anspruch 1, nämlich

H-Gly-Asp-Phe-Glu-DCys-Ile-Pro-Cys-Glu-Tyr-Leu-Gln-OH.

19. Verfahren zur Herstellung eines Peptid-Derivates nach Anspruch 1, nämlich 
(H-Gly-Asp-Phe-Glu-DCys-Ile-Pro-Cys-Glu-Tyr-Leu-Gln-OH)2
|
S
|

|
S
|
 wobei die gezeichneten Bindungen innerhalb des Dimer-Peptids bestehen.

20. Verfahren zur Herstellung eines Peptid-Derivates nach Anspruch 1, nämlich

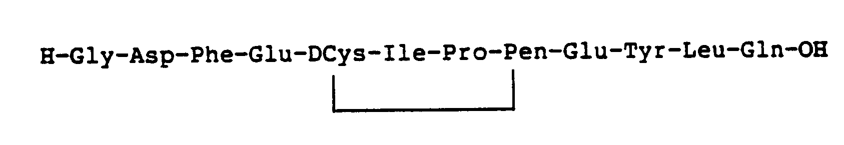
H-Gly-Asp-Phe-Glu-DCys-Ile-Pro-Pen-Glu-Tyr-Leu-Gln-OH

21. Verfahren zur Herstellung eines Peptid-Derivates nach Anspruch 1, nämlich 
(H-Gly-Asp-Phe-Glu-DCys-Ile-Pro-Pen-Glu-Tyr-Leu-Gln-OH)2
                    |           |
                    S           S
                    |           |
 wobei die gezeichneten Bindungen innerhalb des Dimer-Peptids bestehen.

## Patentansprüche (Patentansprüche für folgende(n) Vertragsstaat(en): GR)

1. Peptid-Derivat der Formel 1 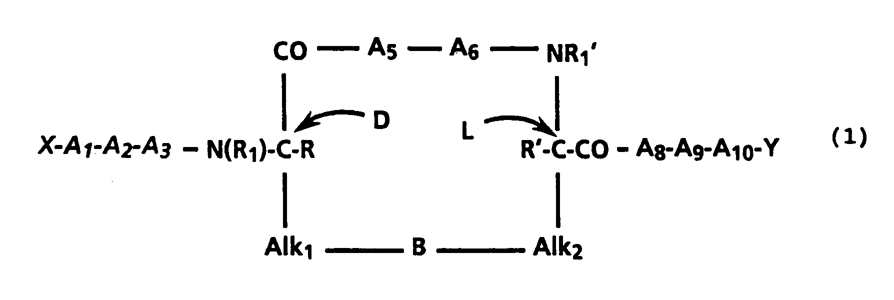 in der
X einen aminoterminalen Rest, ausgewählt aus Wasserstoff, einem oder zwei Alkylresten mit 1 bis 6 Kohlenstoffatomen, einem oder zwei Acylresten mit 2 bis 10 Kohlenstoffatomen, Carbobenzyloxy- oder ter.-Butyloxycarbonylgruppen bedeutet;
A₁ eine Bindung ist, oder ein Peptid mit 1 bis 5 Resten irgendeiner Aminosäure darstellt;
A₂ eine der Gruppen Phe, SubPhe (mono- oder disubstituiertes Phenylalanin), β-(2- und 3-Thienyl)-alanin, β-(2- und 3-Furanyl)-alanin, β-(2-, 3-, und 4-Pyridyl)-alanin, β-(Benzothienyl-2- und 3-yl)-alanin, β-(1- und 2-Naphthyl)-alanin, Tyr oder Trp bedeutet;
A₃ Glu oder Asp ist;
A₅ Ile, Val, Leu, Nle oder Thr bedeutet;
A₆ eine der Gruppen Pro, Hyp, 3,4-DehydroPro, Thiazolidin-4-carboxylat, Sar, NMePgl (N-Methylphenylglycin) oder irgendeine Aminosäure mit D-Konfiguration darstellt;
A₈ Glu oder Asp ist;
A₉ eine lipophile Aminosäure, ausgewählt aus Tyr, Tyr(SO₃H), Trp, Phe, Leu, Nle, Ile, Val, His und Pro bedeutet oder ein mindestens eine dieser lipophilen Aminosäuren enthaltendes Dipeptid ist;
A₁₀ eine Bindung ist oder ein Peptidfragment mit 1 bis 5 Resten irgendwelcher Aminosäuren darstellt;
Y einen carboxyterminalen Rest, ausgewählt aus OH, (C₁-C₆)-Alkoxy, Amino-, Mono- oder Di-(C₁-C₄)alkylsubstituierten Amino- oder Benzylaminogruppen bedeutet;
R, R', R₁ und R₁' jeweils ausgewählt sind aus Wasserstoffatomen oder (C₁-C₄)-Alkylresten;
B ausgewählt ist aus -S-, -S-S- oder -S-Alk₃-S-;
Alk₁, Alk₂ und Alk₃ jeweils ausgewählt sind aus (C₁-C₈)-Methylen- oder Ethylengruppen;
und wobei "D" und "L" anzeigen, daß die Stereochemie am angegebenen Kohlenstoffatom diejenige ist, die dem D-Cystein bzw. L-Cystein entspricht, sowie ein Dimer oder ein Gemisch eines Peptidderivates und des Dimers davon, und ihre pharmazeutisch verträglichen Salze.

2. Peptidderivat nach Anspruch 1, wobei A₂ Phe, β(2 oder 3-Thienyl)-alanin oder Tyr bedeutet.

3. Peptidderivat nach einem der Ansprüche 1 oder 2, wobei A₃ Glu ist.

4. Peptidderivat nach einem der Ansprüche 1 bis 3, wobei A₅ Ile ist.

5. Peptidderivat nach einem der Ansprüche 1 bis 4, wobei A₆ Pro ist.

6. Peptidderivat nach einem der Ansprüche 1 bis 5, wobei A₈ Glu oder Asp ist.

7. Peptidderivat nach einem der Ansprüche 1 bis 6, wobei A₉ Tyr, Ala-Tyr, Ala-Tyr(SO₃H) oder Glu-Leu ist.

8. Peptidderivat nach einem der Ansprüche 1 bis 7, wobei A₁₀ Gln, Leu, Asp, Asp-Glu, Leu-Pro, Leu-Gln, Leu-Ala oder eine Bindung bedeutet.

9. Peptidderivat nach einem der Ansprüche 1 bis 8, wobei X ein Wasserstoffatom, eine Acetyl- oder Succinylgruppe bedeutet.

10. Peptidderivat nach einem der Ansprüche 1 bis 9, wobei Y OH oder NH₂ ist.

11. Peptidderivat nach einem der Ansprüche 1 bis 10, wobei R, R', R₁ und R₁' jeweils ein Wasserstoffatom sind.

12. Peptidderivat nach einem der Ansprüche 1 bis 11, wobei B die Gruppe -S-S- bedeutet.

13. Peptidderivat nach einem der Ansprüche 1 bis 12, wobei Alk₁ und Alk₂ jeweils eine Methylengruppe der Formel -(CH₂)- sind.

14. Peptidderivat nach einem der Ansprüche 1 bis 13, wobei A₁ eine der Gruppen 
His-Asn-Asp-Gly-Asp,
Asn-Asp-Gly-Asp,
Asp-Gly-Asp,
Gly-Asp,
Asp
 oder eine Bindung ist.

15. Verfahren zur Herstellung eines Peptidderivates nach einem der Ansprüche 1 bis 14, eines Dimers davon oder eines Gemisches des Dimeren und des Polypeptids, umfassend die Herstellung des freien sulfhydrylhaltigen linearen Peptids durch Festphasen-Sequenz- oder Blocksynthese, Genclonierung oder eine Kombination davon und anschließend die Durchführung von oxidativer Kupplung mit dem linearen Peptid.

16. Festphasen-Sequenz- oder Blocksyntheseverfahren zur Herstellung eines Peptidderivates nach einem der Ansprüche 1 bis 14, oder eines Dimeren davon oder eines Gemisches des Dimeren und des Polypeptids, umfassend die Bindung einer in geeigneter Weise geschützten Aminosäure der Formel A₁, die die in den vorstehenden Ansprüchen angegebene Bedeutung hat, an einen aktivierten Harzträger, anschließend Bindung der anderen, an der α-Aminogruppe geschützten Aminosäuren der Formeln A₂ bis A₁₀, die die in den vorangehenden Ansprüchen angegebenen Bedeutungen haben, an die endständige Aminogruppe der wachsenden Peptidkette, die in der Zwischenzeit durch Entfernung ihrer Aminoschutzgruppe freigesetzt worden ist, und schließlich Durchführung einer oxidativen Kupplung mit dem linearen Peptid.

17. Peptidderivat nach einem der Ansprüche 1 bis 14 oder ein Dimer davon oder ein Gemisch dieser Verbindungen zur Verwendung als pharmazeutischer Wirkstoff.

18. Peptidderivat nach einem der Ansprüche 1 bis 14 oder ein Dimer davon oder ein Gemisch dieser Verbindungen zur Verwendung als antikoagulierender Wirkstoff.

19. Peptid-Derivat nach Anspruch 1, nämlich

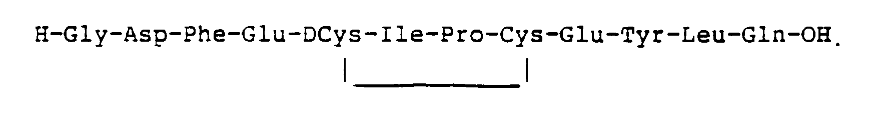
H-Gly-Asp-Phe-Glu-DCys-Ile-Pro-Cys-Glu-Tyr-Leu-Gln-OH.

20. Peptid-Derivat nach Anspruch 1, nämlich 
(H-Gly-Asp-Phe-Glu-DCys-Ile-Pro-Cys-Glu-Tyr-Leu-Gln-OH)2
|
S
|
|
S
|
 wobei die gezeichneten Bindungen innerhalb des Dimer-Peptids bestehen.

21. Peptid-Derivat nach Anspruch 1, nämlich

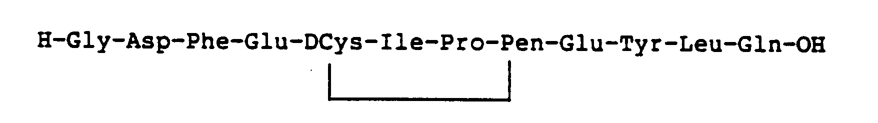
H-Gly-Asp-Phe-Glu-DCys-Ile-Pro-Pen-Glu-Tyr-Leu-Gln-OH

22. Peptid-Derivat nach Anspruch 1, nämlich 
(H-Gly-Asp-Phe-Glu-DCys-Ile-Pro-Pen-Glu-Tyr-Leu-Gln-OH)₂
|
S
|
|
S
|
 wobei die gezeichneten Bindungen innerhalb des Dimer-Peptids bestehen.

## Revendications (Revendications pour l'(les) Etat(s) contractant(s) suivant(s): AT, BE, CH, DE, FR, GB, IT, LI, LU, NL, SE)

1. Un dérivé de peptide de formule 1 : 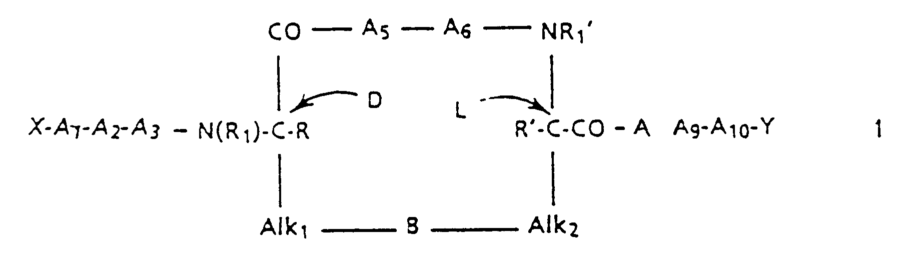 dans laquelle :
X est un résidu terminal du groupe amino choisi parmi les suivants : un atome d'hydrogène, un ou deux groupes alkyles de 1 à 6 atomes de carbone, un ou deux groupes acyles de 2 à 10 atomes de carbone, un groupe carbobenzyloxy ou t-butyloxycarbonyle ;
A₁ est une liaison ou est un peptide contenant de un à cinq résidus d'un aminoacide quelconque ;
A₂ est Phe, subPhe (mono- ou di-substitué phénylalanine), β-(2- et 3-thiényl)alanine, β-(2- et 3-furannyl)alanine, β-(2-, 3-, et 4-pyridyl)alanine, β-(benzothiényl-2- et 3-yl)alanine, β-(1- et 2-naphtyl)alanine, Tyr, ou Trp ;
A₃ est Glu ou Asp ;
A₅ est Ile, Val, Leu, Nle ou Thr ;
A₆ est Pro, Hyp, 3,4-déhydroPro, thiozolidine-4-carboxylate, Sar, NMePgl (N-méthylphénylglycine) ou un aminoacide quelconque ayant la configuration D ;
A₈ est Glu ou Asp ;
A₉ est un aminoacide lipophile choisi parmi les suivants : Tyr, Tyr(SO₃H), Trp, Phe, Leu, Nle, Ile, Val, His, et Pro ou est un dipeptide contenant au moins l'un de ces aminoacides lipophiles ;
A₁₀ est une liaison ou est un fragment de peptide contenant de un à cinq résidus d'un aminoacide quelconque ;
Y est un résidu terminal du groupe carboxy choisi parmi les suivants : OH, (C₁-C₆)alcoxy, amino, mono- ou di-(C₁-C₄)alkyle substitué amino, ou benzylamino ;
R, R', R₁ et R₁' sont chacun choisis parmi l'hydrogène ou un groupe (C₁-C₄)alkyle ;
B est choisi parmi -S-, -S-S-, ou -S-Alk₃-S- ; et
Alk₁, Alk₂, et Alk₃ sont chacun choisis parmi un groupe (C₁-C₈)méthylène ou un groupe éthylène ;
et selon laquelle "D" et "L" indiquent que la stéréochimie du carbone indiqué est celle correspondant à la D-cystéine et à la L-cystéine, respectivement, ainsi qu'un dimère ou un mélange d'un dérivé de peptide et du dimère correspondant et leurs sels pharmaceutiquement acceptables.

2. Un dérivé de peptide selon la revendication 1, selon laquelle A₂ est Phe, β-(2- ou 3-thiényl)alanine, ou Tyr.

3. Un dérivé de peptide selon la revendication 1 ou 2, selon laquelle A₃ est Glu.

4. Un dérivé de peptide selon l'une quelconque des revendications 1 à 3, selon laquelle A₅ est Ile.

5. Un dérivé de peptide selon l'une quelconque des revendications 1 à 4, selon laquelle A₆ est Pro.

6. Un dérivé de peptide selon l'une quelconque des revendications 1 à 5, selon laquelle A₈ est Glu ou Asp.

7. Un dérivé de peptide selon l'une quelconque des revendications 1 à 6, selon laquelle A₉ est Tyr, Ala-Tyr, Ala-Tyr (SO₃H) ou Glu-Leu.

8. Un dérivé de peptide selon l'une quelconque des revendications 1 à 7, selon laquelle A₁₀ est Gln, Leu, Asp, Asp-Glu, Leu-Pro, Leu-Gln, Leu-Ala, ou une liaison.

9. Le dérivé de peptide selon l'une quelconque des revendications 1 à 8, selon laquelle X est H, acétyle ou succinyle.

10. Le dérivé de peptide selon l'une quelconque des revendications 1 à 9, selon laquelle Y est OH ou NH₂.

11. Le dérivé de peptide selon l'une quelconque des revendications 1 à 10, selon laquelle R, R', R₁, R₁' désignent chacun un atome d'hydrogène.

12. Le dérivé de peptide selon l'une quelconque des revendications 1 à 11, selon laquelle B est le groupe -S-S-.

13. Le dérivé de peptide selon l'une quelconque des revendications 1 à 12, selon laquelle Alk₁ et Alk₂ désignent chacun un groupe méthylène de formule -(CH₂)-.

14. Le dérivé de peptide selon l'une quelconque des revendications 1 à 13, selon laquelle

A1 est His–Asn–Asp–Gly–Asp,
Asn–Asp–Gly–Asp,
Asp–Gly–Asp,
Gly–Asp,
Asp, ou une liaison.

15. Un procédé de préparation d'un dérivé de peptide selon l'une quelconque des revendications 1 à 14, d'un dimère de ce dérivé, ou d'un mélange du dimère et dudit polypeptide comprenant la préparation du peptide linéaire contenant un groupe sulfhydryle libre par synthèse séquentielle ou en bloc en phase solide, par clonage de gêne, ou une combinaison de ces procédés, et le traitement subséquent du peptide linéaire par couplage oxydant.

16. Procédé de synthèse séquentielle ou en bloc en phase solide pour la préparation d'un dérivé de peptide selon l'une quelconque des revendications 1 à 14, ou d'un dimère du dérivé, ou d'un mélange du dimère et du polypeptide comprenant la liaison d'un aminoacide protégé de façon appropriée de formule A₁ ayant la signification donnée dans les revendications précédentes à un support de résine activée, la liaison subséquente des autres aminoacides à un groupe α-amino protégé de formules A₂ à A₁₀ ayant les significations données dans les revendications précédentes à un groupe amino terminal de la chaîne peptidique en croissance et qui, entre temps, a été exposé pour l'élimination de son groupe protecteur du groupe amino, et finalement le traitement du peptide linéaire par un couplage oxydant.

17. Un dérivé de peptide selon l'une quelconque des revendications 1 à 14, ou un dimère de ce dérivé, ou un mélange de ces composés à utiliser comme substance pharmaceutiquement active.

18. Un dérivé de peptide selon l'une quelconque des revendications 1 à 14, ou un dimère de ce dérivé, ou un mélange de ces composés à utiliser comme anticoagulant.

19. Une composition pharmaceutique contenant une quantité efficace comme anticoagulant d'un dérivé de peptide selon l'une quelconque des revendications 1 à 14, ou d'un dimère de ce dérivé, ou d'un mélange de ces composés, et éventuellement un support et/ou un diluant pharmaceutiquement acceptable.

20. Un dérivé de peptide selon la revendication 1 qui est

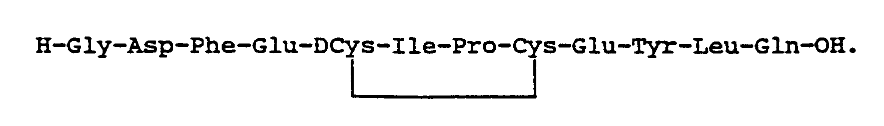
H-Gly-Asp-Phe-Glu-DCys-Ile-Pro-Cys-Glu-Tyr-Leu-Gln-OH.


21. Un dérivé de peptide selon la revendication 1 qui est 
(H-Gly-Asp-Phe-Glu-DCys-Ile-Pro-Cys-Glu-Tyr-Leu-Gln-OH)2,
S S
 où les liaisons indiquées se produisent dans le peptide dimère.

22. Un dérivé de peptide selon la revendication 1 qui est

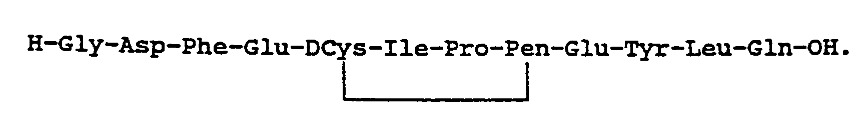
H-Gly-Asp-Phe-Glu-DCys-Ile-Pro-Pen-Glu-Tyr-Leu-Gln-OH.

23. Un dérivé de peptide selon la revendication 1 qui est 
(H-Gly-Asp-Phe-Glu-DCys-Ile-Pro-Pen-Glu-Tyr-Leu-Gln-OH)2,
                    |              |
                    S              S
                    |              |
 où les liaisons indiquées se produisent dans le peptide dimère.

## Revendications (Revendications pour l'(les) Etat(s) contractant(s) suivant(s): ES)

1. Un procédé de préparation d'un dérivé de peptide de formule : 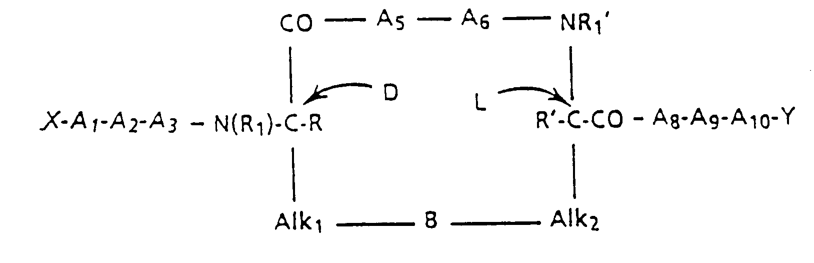 dans laquelle :
X est un résidu terminal du groupe amino choisi parmi les suivants : un atome d'hydrogène, un ou deux groupes alkyles de 1 à 6 atomes de carbone, un ou deux groupes acyles de 2 à 10 atomes de carbone, un groupe carbobenzyloxy ou t-butyloxycarbonyle ;
A₁ est une liaison ou est un peptide contenant de un à cinq résidus d'un aminoacide quelconque ;
A₂ est Phe, subPhe (mono- ou di-substitué phénylalanine), β-(2- et 3-thiényl)alanine, β-(2- et 3-furannyl)alanine, β-(2-, 3-, et 4-pyridyl)alanine, β-(benzothiényl-2- et 3-yl)alanine, β-(1- et 2-naphtyl)alanine, Tyr, ou Trp ;
A₃ est Glu ou Asp ;
A₅ est Ile, Val, Leu, Nle ouThr ;
A₆ est Pro, Hyp, 3,4-déhydroPro, thiazolidine-4-carboxylate, Sar, NMePgl (N-méthylphénylglycine), ou un aminoacide quelconque ayant la configuration D ;
A₈ est Glu ou Asp ;
A₉ est un aminoacide lipophile choisi parmi les suivants : Tyr, Tyr(SO₃H), Trp, Phe, Leu, Nle, Ile, Val, His, et Pro ou est un dipeptide contenant au moins l'un de ces aminoacides lipophiles ;
A₁₀ est une liaison ou est un fragment de peptide contenant de un à cinq résidus d'un aminoacide quelconque ;
Y est un résidu terminal du groupe carboxy choisi parmi les suivants : OH, (C₁-C₆)alcoxy, amino, mono- ou di-(C₁-C₄)alkyle substitué amino, ou benzylamino ;
R, R', R₁ et R₁' sont chacun choisis parmi l'hydrogène ou un groupe (C₁-C₄)alkyle ;
B est choisi parmi -S-, -S-S-, ou -S-Alk₃-S- ; et
Alk₁, Alk₂, et Alk₃ sont chacun choisis parmi un groupe (C₁-C₈)méthylène ou un groupe éthylène ;
et selon laquelle "D" et "L" indiquent que la stéréochimie du carbone indiqué est celle correspondant à la D-cystéine et à la L-cystéine, respectivement, ainsi qu'un dimère ou un mélange d'un dérivé de peptide et du dimère correspondant, comprenant la préparation du peptide linéaire contenant un groupe sulfhydryle libre par synthèse séquentielle ou en bloc en phase solide, par clonage de gêne, ou par une combinaison de ces méthodes et le traitement subséquent du peptide linéaire par un couplage oxydant.

2. Un procédé de synthèse séquentiel ou en bloc en phase solide pour la préparation d'un dérivé de peptide de formule : 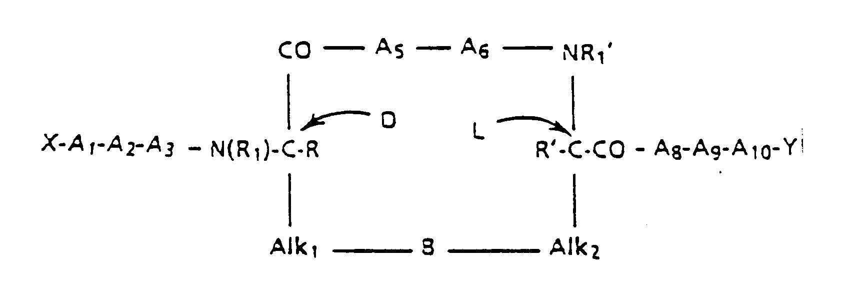 dans laquelle :
X est un résidu terminal du groupe amino choisi parmi les suivants : un atome d'hydrogène, un ou deux groupes alkyles de 1 à 6 atomes de carbone, un ou deux groupes acyles de 2 à 10 atomes de carbone, un groupe carbobenzyloxy ou t-butyloxycarbonyle ;
A₁ est une liaison ou est un peptide contenant de un à cinq résidus d'un aminoacide quelconque ;
A₂ est Phe, subPhe, β-(2- et 3-thiényl)alanine, β-(2- et 3-furannyl)alanine, β-(2-, 3-, et 4-pyridyl)alanine, β-(benzothiényl-2- et 3-yl)alanine, β-(1- et 2-naphtyl)alanine, Tyr, ou Trp ;
A₃ est Glu ou Asp ;
A₅ est Ile, Val, Leu, Nle ou Thr ;
A₆ est Pro, Hyp, 3,4-déhydroPro, thiazolidine-4-carboxylate, Sar, NMePgl, ou un aminoacide quelconque ayant la configuration D ;
A₈ est Glu ou Asp ;
A₉ est un aminoacide lipophile choisi parmi les suivants : Tyr, Tyr(SO₃H), Trp, Phe, Leu, Nle, Ile, Val, His, et Pro ou est un dipeptide contenant au moins l'un de ces aminoacides lipophiles ;
A₁₀ est une liaison ou est un fragment de peptide contenant de un à cinq résidus d'un aminoacide quelconque ;
Y est un résidu terminal du groupe carboxy choisi parmi les suivants : OH, (C₁-C₆)alcoxy, amino, mono- ou di-(C₁-C₄)alkyle substitué amino, ou benzylamino ;
R, R', R₁ et R₁' sont chacun choisis parmi l'hydrogène ou un groupe (C₁-C₄)alkyle ;
B est choisi parmi -S-, -S-S-, ou -S-Alk₃-S- ; et
Alk₁, Alk₂, et Alk₃ sont chacun choisis parmi un groupe (C₁-C₈)méthylène ou un groupe éthylène ;
et selon laquelle "D" et "L" indiquent que la stéréochimie du carbone indiqué est celle correspondant à la D-cystéine et à la L-cystéine, respectivement, ainsi qu'un dimère ou un mélange d'un dérivé de peptide et du dimère correspondant, comprenant la liaison d'un aminoacide protégé de façon appropriée de formule A₁ à un support de résine activé, la liaison subséquente des autres aminoacides à groupe α-amino protégé de A₂ à A₁₀ à un groupe amino terminal de la chaîne peptidique en croissance qui a été entre temps exposé pour l'élimination de son groupe protecteur du groupe amino, et finalement le traitement du peptide linéaire par un couplage oxydant.

3. Un procédé selon la revendication 1 ou 2, selon laquelle A₂ est Phe, β-(2- ou 3-thiényl)alanine, ou Tyr.

4. Un procédé selon l'une quelconque des revendications 1 à 3, selon laquelle A₃ est Glu.

5. Un procédé selon l'une quelconque des revendications 1 à 4, selon laquelle A₅ est Ile.

6. Un procédé selon l'une quelconque des revendications 1 à 5, selon laquelle A₆ est Pro.

7. Un procédé selon l'une quelconque des revendications 1 à 6, selon laquelle A₈ est Glu ou Asp.

8. Un procédé selon l'une quelconque des revendications 1 à 7, selon laquelle A₉ est Tyr, Ala-Tyr, Ala-Tyr(SO₃H) ou Glu-Leu.

9. Un procédé selon l'une quelconque des revendications 1 à 8, selon laquelle A₁₀ est Gln, Leu, Asp, Asp-Glu, Leu-Pro, Leu-Gln, Leu-Ala, ou une liaison.

10. Un procédé selon l'une quelconque des revendications 1 à 9, selon laquelle X est H, acétyle ou succinyle.

11. Un procédé selon l'une quelconque des revendications 1 à 10, selon laquelle Y est OH ou NH₂.

12. Un procédé selon l'une quelconque des revendications 1 à 11, selon laquelle R, R', R₁, R₁' désignent chacun un atome d'hydrogène.

13. Un procédé selon l'une quelconque des revendications 1 à 12, selon laquelle B est le coupe -S-S-.

14. Un procédé selon l'une quelconque des revendications 1 à 13, selon laquelle Alk₁ et Alk₂ désignent chacun un groupe méthylène de formule -(CH₂)-.

15. Un procédé selon l'une quelconque des revendications 1 à 14, selon laquelle A₁ est :

His–Asn–Asp–Gly–Asp,
Asn–Asp–Gly–Asp,
Asp–Gly–Asp,
Gly–Asp,
Asp, ou une liaison.

16. Utilisation d'un composé obtenu selon le procédé de l'une quelconque des revendications 1 à 15, pour la préparation d'une composition pharmaceutique.

17. Utilisation d'un composé obtenu selon le procédé de l'une quelconque des revendications 1 à 15, pour la préparation d'un anticoagulant.

18. Un procédé selon la revendication 1 pour la préparation d'un dérivé de peptide qui est

H-Gly-Asp-Phe-Glu-DCys-Ile-Pro-Cys-Glu-Tyr-Leu-Gln-OH.

19. Un procédé selon la revendication 1 pour la préparation d'un dérivé de peptide qui est 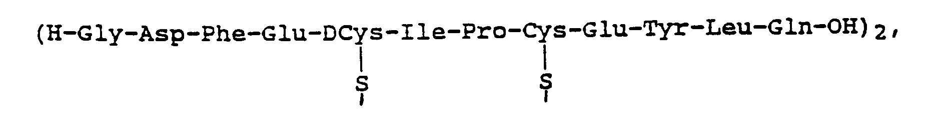
(H-Gly-Asp-Phe-Glu-DCys-Ile-Pro-Cys-Glu-Tyr-Leu-Gln-OH)2,
S S
 où les liaisons indiquées se produisent dans le peptide dimère.

20. Un procédé selon la revendication 1 pour la préparation d'un dérivé de peptide qui est

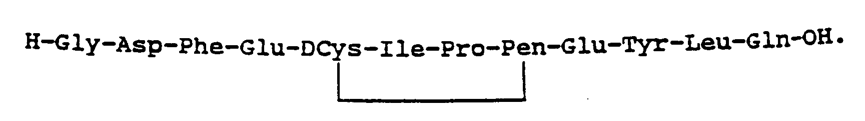
H-Gly-Asp-Phe-Glu-DCys-Ile-Pro-Pen-Glu-Tyr-Leu-Gln-OH.

21. Un procédé selon la revendication 1 pour la préparation d'un dérivé de peptide qui est 
(H-Gly-Asp-Phe-Glu-DCys-Ile-Pro-Pen-Glu-Tyr-Leu-Gln-OH)2,
|
S
|
|
S
|
 où les liaisons indiquées se produisent dans le peptide dimère.

## Revendications (Revendications pour l'(les) Etat(s) contractant(s) suivant(s): GR)

1. Un dérivé de peptide de formule 1 : 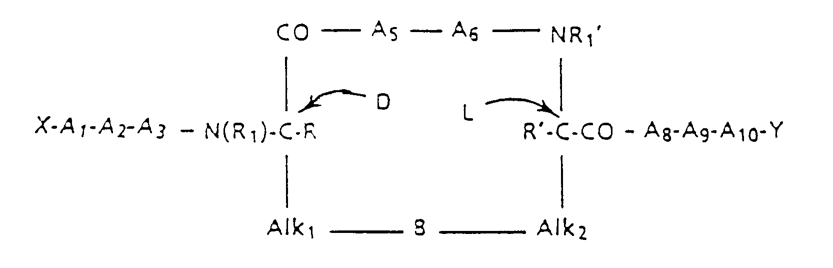 dans laquelle :
X est un résidu terminal du groupe amino choisi parmi les suivants : un atome d'hydrogène, un ou deux groupes alkyles de 1 à 6 atomes de carbone, un ou deux groupes acyles de 2 à 10 atomes de carbone, un groupe carbobenzyloxy ou t-butyloxycarbonyle ;
A₁ est une liaison ou est un peptide contenant de un à cinq résidus d'un aminoacide quelconque ;
A₂ est Phe, subPhe (mono- ou di-substitué phénylalanine), β-(2- et 3-thiényl)alanine, β-(2- et 3-furannyl)alanine, β-(2-, 3-, et 4-pyridyl)alanine, β-(benzothiényl-2- et 3-yl)alanine, β-(1- et 2-naphtyl)alanine, Tyr, ou Trp ;
A₃ est Glu ou Asp ;
A₅ est Ile, Val, Leu, Nle ou Thr;
A₆ est Pro, Hyp, 3,4-déhydroPro, thiazolidine-4-carboxylate, Sar NMePgl (N-méthylphénylglycine), ou un aminoacide quelconque ayant la configuration D ;
A₈ est Glu ou Asp ;
A₉ est un aminoacide lipophile choisi parmi les suivants : Tyr, Tyr(SO₃H), Trp, Phe, Leu, Nle, Ile, Val, His, et Pro ou est un dipeptide contenant au moins l'un de ces aminoacides lipophiles ;
A₁₀ est une liaison ou est un fragment de peptide contenant de un à cinq résidus d'un aminoacide quelconque ;
Y est un résidu terminal du groupe carboxy choisi parmi les suivants : OH, (C₁-C₆)alcoxy, amino, mono- ou di-(C₁-C₄)alkyle substitué amino, ou benzylamino ;
R, R', R₁ et R₁' sont chacun choisis parmi l'hydrogène ou un groupe (C₁-C₄)alkyle ;
B est choisi parmi -S-, -S-S-, ou -S-Alk₃-S- ; et
Alk₁, Alk₂, et Alk₃ sont chacun choisis parmi un groupe (C₁-C₈)méthylène ou un groupe éthylène ;
et selon laquelle "D" et "L" indiquent que la stéréochimie du carbone indiqué est celle correspondant à la D-cystéine et à la L-cystéine, respectivement, ainsi qu'un dimère ou un mélange d'un dérivé de peptide et du dimère correspondant et leurs sels pharmaceutiquement acceptables.

2. Un dérivé de peptide selon la revendication 1, selon laquelle A₂ est Phe, β-(2- ou 3-thiényl)alanine, ou Tyr.

3. Un dérivé de peptide selon la revendication 1 ou 2, selon laquelle A₃ est Glu.

4. Un dérivé de peptide selon l'une quelconque des revendications 1 à 3, selon laquelle A₅ est Ile.

5. Un dérivé de peptide selon l'une quelconque des revendications 1 à 4, selon laquelle A₆ est Pro.

6. Un dérivé de peptide selon l'une quelconque des revendications 1 à 5, selon laquelle A₈ est Glu ou Asp.

7. Un dérivé de peptide selon l'une quelconque des revendications 1 à 6, selon laquelle A₉ est Tyr, Ala-Tyr, Ala-Tyr (SO₃H) ou Glu-Leu.

8. Un dérivé de peptide selon l'une quelconque des revendications 1 à 7, selon laquelle A₁₀ est Gln, Leu, Asp, Asp-Glu, Leu-Pro, Leu-Gln, Leu-Ala, ou une liaison.

9. Le dérivé de peptide selon l'une quelconque des revendications 1 à 8, selon laquelle X est H, acétyle ou succinyle.

10. Le dérivé de peptide selon l'une quelconque des revendications 1 à 9, selon laquelle Y est OH ou NH₂.

11. Le dérivé de peptide selon l'une quelconque des revendications 1 à 10, selon laquelle R, R', R₁, R₁' désignent chacun un atome d'hydrogène.

12. Le dérivé de peptide selon l'une quelconque des revendications 1 à 11, selon laquelle B est le groupe -S-S-.

13. Le dérivé de peptide selon l'une quelconque des revendications 1 à 12, selon laquelle Alk₁ et Alk₂ désignent chacun un groupe méthylène de formule -(CH₂)-.

14. Le dérivé de peptide selon l'une quelconque des revendications 1 à 13, selon laquelle

A1 est His–Asn–Asp–Gly–Asp
Asn–Asp–Gly–Asp,
Asp–Gly–Asp,
Gly–Asp,
Asp, ou une liaison.

15. Un procédé de préparation d'un dérivé de peptide selon l'une quelconque des revendications 1 à 14, d'un dimère de ce dérivé, ou d'un mélange du dimère et dudit polypeptide comprenant la préparation du peptide linéaire contenant un groupe sulfhydryle libre par synthèse séquentielle ou en bloc en phase solide, par clonage de gêne, ou une combinaison de ces procédés, et le traitement subséquent du peptide linéaire par couplage oxydant.

16. Procédé de synthèse séquentielle ou en bloc en phase solide pour la préparation d'un dérivé de peptide selon l'une quelconque des revendications 1 à 14, ou d'un dimère du dérivé, ou d'un mélange du dimère et du dipolypeptide comprenant la liaison d'un aminoacide protégé de façon appropriée de formule A₁ ayant la signification donnée dans les revendications précédentes à un support de résine activée, la liaison subséquente des autres aminoacides à groupe α-amino protégé de formules A₂ à A₁₀ ayant les significations données dans les revendications précédentes à un groupe amino terminal de la chaîne peptidique en croissance et qui, entre temps, a été exposé pour l'élimination de son groupe protecteur du groupe amino, et finalement le traitement du peptide linéaire par un couplage oxydant.

17. Un dérivé de peptide selon l'une quelconque des revendications 1 à 14, ou un dimère de ce dérivé, ou un mélange de ces composés à utiliser comme composés pharmaceutiquement actifs.

18. Un dérivé de peptide selon l'une quelconque des revendications 1 à 14, ou un dimère de ce dérivé, ou un mélange de ces composés à utiliser comme anticoagulant.

19. Un dérivé de peptide selon la revendication 1 qui est

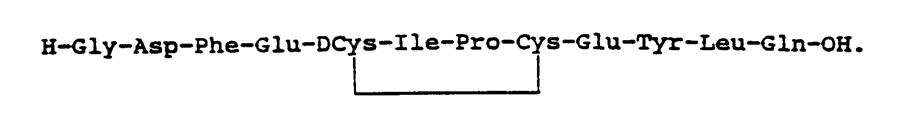
H-Gly-Asp-Phe-Glu-DCys-Ile-Pro-Cys-Glu-Tyr-Leu-Gln-OH.

20. Un dérivé de peptide selon la revendication 1 qui est 
(H-Gly-Asp-Phe-Glu-DCys-Ile-Pro-Cys-Glu-Tyr-Leu-Gln-OH)2,
S S
 où les liaisons indiquées se produisent dans le peptide dimère.

21. Un dérivé de peptide selon la revendication 1 qui est

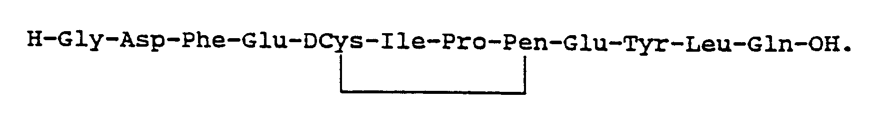
H-Gly-Asp-Phe-Glu-DCys-Ile-Pro-Pen-Glu-Tyr-Leu-Gln-OH.


22. Un dérivé de peptide selon la revendication 1 qui est 
(H-Gly-Asp-Phe-Glu-DCys-Ile-Pro-Pen-Glu-Tyr-Leu-Gln-OH)2,
                    |               |
                    S               S
                    |               |
 où les liaisons indiquées se produisent dans le peptide dimère.
